# EUROPEAN PATENT SPECIFICATION

(11) **EP 1 522 307 B1**
(45) Date of publication and mention of the grant of the patent: **04.08.2010**
(21) Application number: 03707241.0
(22) Date of filing: 26.02.2003
(51) Int. Cl.: A61K 35/14, A61K 35/26, A61K 35/28, A61P 31/00

(54) **DIALYZED LEUKOCYTE EXTRACT FOR THE TREATMENT OF INFECTIOUS DISEASES IN ANIMALS**
DIALYSIERTER LEUKOZYTENEXTRAKT ZUR BEHANDLUNG VON INFEKTIONSKRANKHEITEN BEI TIEREN
EXTRAIT DE LEUCOCYTES DIALYSE POUR LE TRAITEMENT DE MALADIES INFECTIEUSES CHEZ DES ANIMAUX

(30) Priority: 28.02.2002 MX 0202171
(43) Date of publication of application: 13.04.2005
(73) Proprietor: Calzada Nova, Luis Antonio, 04870 Mexico, D.F. (MX)
(72) Inventor: Calzada Nova, Luis Antonio, 04870 Mexico, D.F. (MX)
(74) Representative: Predazzi, Valentina
(86) International application number: PCT/MX2003/000016
(87) International publication number: WO 2003/072117

(56) References cited:
- EP-A1- 0 109 089
- WO-A1-80/00790
- US-A- 3 991 182
- US-A- 4 132 776
- US-A- 5 470 835
- MAZAHERI M.R.: 'Immunotherapy of viral infections with transfer factor' JOURNAL OF MEDICAL VIROLOGY vol. 1, no. 3, 1977, pages 209 - 217, XP002972519
- DATABASE BIOSIS [Online] LYUBCHENKO T.A. ET AL.: 'Biological activity of transfer factor induced by bacterial antigens', XP002971704 Database accession no. (PREV199800049042) & MIKROBIOLOHICHNYI ZHURNAL 1997,
- DATABASE MEDLINE [Online] SMOGORZEWSKA E.: 'Transfer of delayed type hypersensitivity in guinea pigs', XP002971705 Database accession no. (NLM2152405) & PROBLEMY MEDYCYNY WIEKU ROZWOJOWEGO 1990, POLONIA,

## Description

### Field of invention.

The present invention is related to the field of treatment of infectious diseases by means of the transference and stimulation of immunity by cells through dialyzed leukocyte extract, as well as methods of obtaining and preserving the extract.

### Background of the invention.

Diseases always have been part of the experience of human life and animal. From the establishment of bases for the prevention and treatment of the infections effective therapies against most of the diseases have been implemented.

In spite of all the advances in the treatment of diseases in animals, it doesn't exist until before the present invention, effective therapeutic treatments against certain types, such as those in which the prognosis is not much favorable for the animal after the typical signs appear, with which the patient has few probabilities of surviving. In these cases, the treatment had been limited to try to preserve the animal in the best possible physiological state to allow that its own immunological system was able to respond. Examples of these diseases are Aujesky's disease and canine distemper.¹

Canine distemper represents to date, one of the most important domestic diseases and of world-wide dissemination.

Canine distemper is a systemic, infectious disease, immunosuppressive and highly contagious, of acute or sub acute evolution, with respiratory, gastrointestinal and neurological clinical signs. This illness has a high index of mortality that affects dogs and other carnivores around the world and it's transmitted by a viral agent. Is the most prevalent viral disease in dogs and it's considered that there're few in the world that live in isolated zones and that have not been exposed or infected with the virus.

Infection occurs mainly by aerosol spread or direct contact with infected dogs, replicating itself in the systemic lymphoid tissues, causing fever, viremia and dissemination to epithelial tissues and the central nervous system; Evolution depends on the amount of inoculum, the viral stock and the immunological answer of the guest. Disease presents multi systemic signs, fever and a high percentage of mortality; once the clinical signs characteristic of canine distemper become evident, the prognosis is extremely poor, most of them progress of unfavorable way, independently of the treatment and the periods of apparent improvement; as consequence, animals die with or without neurological signs.

Canine distemper is the infect-contagious disease with the highest indices of morbidity in dogs, which oscillates between the 25 and 50%. Mortality is as high as from the 50 to 100% of the cases depending on the viral stock, the type of affected canine population, age of dogs, state of population immunization and the immunological answer of animals. Rabies is the only disease that has an index of higher mortality to the one of canine distemper, since this is always of 100%.

Before the present invention, specific treatment for this one disease didn't exist. The treatment consisted solely of quimiotherapeutic drug administration, in such a way that the treatment was limited to restoration of symptomatic actions and euthanasia once neurological signs were observed. During disease's evolution, it's possible happens the spontaneous recovery in some dogs with no nervous systemic canine distemper, with the application of symptomatic therapeutic actions, which can grant unsuitable credits to certain therapeutic regimens.^{2,3,4,5,6,7}

Like in canine distemper, in diverse infect-contagious diseases an important affectation in the answer of the immunological system is observed, which allows a superior evolution of disease.

Among factors that can cause or favor immunodeficiency in animals, are the serious undernourishment, cytotoxic chemotherapy, over population, as well as any other cause that causes intense or prolonged stress, to mention some. Immune suppression generates several effects, such as a superior frequency of infections of severe type, parasitic, bacterial and viral infections, persistent or recurrent, with modest or no answer to the appropriate treatment and the infection with nonpathogenic microorganisms. Exhaustive studies in relation to the process of immune system in animals have been made and the playing role immune system takes in organisms to fight infections. Several lines of investigation has been studied in this one sense, the study of the immunity transference from an organism to another one through diverse strategies, which mainly involve fluid or cells in the immune system.

Landsteiner and Chase described the immunity transference which could be made through cells of sensitized guinea pigs donors to no sensitized guinea pigs receptors, when inducing allergies by contact to picryl chloride injecting peritoneal exudate cells from sensitized animals in the skin of animal receptors. Later they demonstrated that the intraskin injection of isolated leukocytes of peritoneal exudate induced in sensitized guinea pigs to tuberculin can produce "delayed" specific cutaneous hypersensitivity to tuberculin in not sensitized guinea pigs. In that same work it was tried to make the transference of this reactivity using serum and died cells, without satisfactory results. Later it was determined that under certain conditions it was possible to transfer hypersensitivity by contact of sensitized guinea pigs with antigens of poison ivy to no sensitized guinea pigs receptors, by means of the injection of died lymphoid cells. Also in later experiments similar results were obtained in the transference of cellular immunity by means of the use of dinitrobencencloride as antigen and leukocyte's extracts as vehicle of transference.

Lawrence reveals that the intraskin injection of viable leukocytes obtained of positive human donors to the intradermal reaction with tuberculin in negative people to the antigen mentioned one, can induce passive cutaneous hypersensitivity; also mentions that transferred cutaneous hypersensitivity is a transient with duration of four days to three months.⁸ Later it confirms that the degree of duration of the transferred sensitive state keeps a relation with the degree from sensibilitation of the donating leukocyte and the amount of used leukocytes; also, that the delayed sensitivity induced after the injection of leukocyte lysates is similar to the observed one with the injection of viable leukocytes.⁹ Until that moment the meaning of transference of cutaneous hypersensitivity was unknown and consequently these works received modest attention. Results obtained by Lawrence, related to the dialyzed leukocyte extract (DLE) were perceived with skepticism by some immunologists and alternative explanations seted out. One was based on the point of which Lawrence in its experiments always used infectious disease antigens or common vaccine components and that the receiving organism reacted by primary sensibilitation; also one seted out that receiving contents in the dialyzed leukocyte extracts already were sensitized to the antigens and the dialyzed extract acted like booster or intensifier of preexisting sensitivities. In subsequent experiments with synthetic antigens and controlled studies made with mice it was possible to reject these possibilities. Also it was postulated that the dialyzed one contained fragments of highly immunogenic antigens and that the dialyzed leukocyte extract produced its effects by active sensibilitation of receptors, theory that to date has been rejected.

In 70's the interest by DLE resurges and the transfer factor on it contained, when it was observed it's application in people with certain immunodeficiency syndromes of genetic origin, like the Wiskott-Aldrich syndrome, improved the clinical and immunological parameters. With it the clinical investigation began with the application of dialyzed leukocyte extract.¹⁰

In the clinical area of human medicine the use of DLE as treatment of different diseases has been described, such as infections by herpes simplex virus smallpox¹¹, measles, dermatological affections of the type of melasma, acne, condilomatosis and papilomatosis.¹² Also it has shown effectiveness against infections by *Candida albicans*,¹³ *Varicella zoster,*¹⁴ *Cryptosporidium spp,*^{15,16} *Mycobacterium tuberculosis*¹⁷ *and Actinobacillus pleuropneumoniae.*

In animals the transfer of delayed hypersensitivity in diverse diseases has been described. For the case of pigs in colibacilosis, Aujesky's disease, pig cholera and transmissible gastroenteritis; in bovines in Infectious Rinotraqueitis, salmonellosis and paratuberculosis; in chickens in Newcastle's disease, infectious Laryngotracheitis, Marek's Disease and coccidiosis; and in rabbits in infectious cold.

However, in multiple studies made in animals and human beings for therapeutic aims using dialyzed extract, a great heterogeneity in the obtained results has been observed, since treatments are not reported that work in a consisting way in a high percentage of the patients and in which a complete recovery health state is observed, so is the case of infections caused by *Candida albicans,***^{18,19}** *Leismania* and *Salmonella.*²⁰ In some cases, extract application to patients with chronic or recurrent infections has been made like a last effort in patients in which treatments have failed without obtaining positive results in the recovery, even being observed death.²¹

According to reports until today, success of such therapies depends on multiple factors, from the health condition of patient (including the stage of evolution of the disease), disease in himself, and even source from which leukocytes extract²² was obtained in this one sense, the previous sensibilitation of donor leukocytes to the involved specific antigens in disease that will be treat in patients, is a determining factor for the observation of therapeutic effects.^{23,24,34}

With relation to leukocyte extract, later studies allowed to determine some of their properties, which located the extract as dialyzable, heat-stable, resistant to freezing and treatment with DNase, RNAse or trypsin²⁵ that contained molecules of a molecular weight smaller than 10,000 Daltons²⁶ adenine, guanine, cytosine, uracil and ribophosphate in polynucleotide material^{27,28} and small polypeptides.

Later it was determined that the extract contains more than 200 different molecules with molecular weights from 1,000 to 12,000 Daltons of which it has been possible to identify some (nicotinamide, serotonin, ascorbate, timosin, prostaglandins, interleukin-8, transfer factor, hypoxanthine and uracil, among others). Also, until today it has been possible to determine and to isolate some of these molecules as well as fractions of extract and to study it's role in the therapeutic effects that have been observed derivative of their application, such as a denominated fraction "immune peptide"**²⁹** (responsible for the formation of rosettes of T cells with erythrocytes of sheep), fraction B consistent of twelve amino acids and three to four bases of RNA**³⁰** (responsible for the effect of delayed hypersensitivity to no immune receptors), fraction Tx**³¹** (able to transfer delayed hypersensitivity to guinea pigs) and highly polar peptides, hydrophilic and with a very powerful biological activity, like for example LLYAQDLEDN and LLYAQDVEDN.**³²**

On the other hand it has been reported that dialyzed leukocyte extract presents a medium stability, conserving itself solely its therapeutic effects by several months to temperatures of -20°C to -70°C.³⁴ These conservation methods of extract are not manageable, even in situations of administration to field patients (in farms or countryside where apparatuses of freezing to these temperatures do not exist) as well as for its sale and distribution to public.

In later studies, it was managed to increase its stability to storage to 4°C by means of addition of bovine seric albumin to the extract³³ with which substances are added that can cause adverse effects to patient (disease transmission either increase of immune responses to saver) or make more susceptible to contamination the extract.

In relation to its therapeutic effects, it is known that dialyzed leukocyte extract has many effects on cellular immunity since it respectively contains inductive factors and suppressors obtained of t-suppressors and t-aids cells. It has been argued that the variety of biological activities observed, it is derived form an heterogeneous molecule mixture that extract contains, being each one of them responsible for one or several effects. The answers and effects observed in clinical applications of extract have allowed to locate it like a useful immunomodulator for immunotherapy and immunoprophylaxis of diseases in where cellular immunity is gotten depressed of primary or secondary form, as well as in morbid states in where immunity is exacerbated, inducing injuries by autoimmunity.

It has been observed that dialyzed leukocyte extract transfers local and systemic hypersensitivity and doesn't cause incompatible reaction even between diverse species, transfers halo graft rejections, exerts a direct action on production of interleukin 1, 8 and 12, produce an increase of activation of macrophages, increases synthesis of dependent antigen/DNA of lymphocytes, resynthesize receptor of transfer factor, promotes monocytes and neutrophiles quimiotaxis, increases activity of natural killer cells (NK), accelerates formation of rosettes of T cells, induces the formation of lymphocytes populations that react specifically to an antigen, expands lymphocytes populations and cellular immunity, increases lymphokines production and increases cytotoxicity of T cells against specific tumoral antigens; it hasn't been discarded, the possibility that it causes other possible effects.³⁴

To date, the mechanism of action of DLE is not known in which all effects observed in their clinical applications can be explained.

### Detailed description of the invention.

The present invention uses DLE as the active principle of a medication that allows to treat and to prevent diverse infectious animal diseases, preferably those diseases that affect canine, equines and porcine, like for example dogs, horses and pigs.

This invention is sustained in documentary antecedents of DLE in humans as in animals, with the difference that it is used with therapeutic aims, never described in Veterinary Medicine. Test in an ample range of dogs, horses and pigs diseases with reduced doses that optimize their use, obtained from different tissues and administered via parenteral with DLE from its own original tissue different from receiving species. Agreed to the previously described, it is why one of the objectives of the present invention is to provide in animals an effective treatment against infected-contagious diseases with high degree of morbidity in advanced stages by the application of dialyzed leukocytes extract, since until before the present invention, this treatment didn't exist. An additional objective of present invention is to provide in animals an effective treatment against infected-contagious diseases that depress the immune system by means of administration of dialyzed leukocyte extract.

An additional objective of present invention is to provide in dogs an effective treatment against canine distemper, disease with high degree of morbidity in advanced stages, by means of application of DLE, since until before present invention, it wasn't known effective treatments to allowed to restore health of dogs after appearance of clinical signs of the disease.

Here is another one of the objectives of present invention, to provide a method of obtaining DLE from lymph nodes, spleen and thymus with greater yields to known methods to date.

Here is another one of the objectives of present invention, to provide a stable DLE to 4°C without the use of conservatives or savers with which their therapeutic properties are conserved, since until before present invention dialyze stable leukocytes extracts to this one temperature and without use of extraordinary substances to the extract had not been obtained. This is achieved by ozone sterilization of the DLE.

Another objective of the present invention is to provide a therapeutic kit containing DLE for animal treatment affected by infected-contagious diseases with high degree of morbidity in advanced stages.

DLE of present work can be used for treatment of diseases associated to immunodepression, such as the following ones, which are solely to illustrate the specter of application without limiting their use to other diseases or other animals:
- In bovines:: Infectious bovine rinotraqueitis (αherpes virus), Ulcerative mammillitis (α herpes virus), maligned catarrhal fiber (α herpes virus), Aujesky's disease (α herpesvirus), maligned catarrhal fiber (γ herpesvirus), bovine plague (morbillivirus), parainfluenza-3 (morbillibirus), bovine sincitial virus (pneumovirus), neonative diarrea of the calf (coronavirus), papilomatosis (papilomavirus), bovine leucosis (oncornavirus), hemorrhagic septicemia (*Pasteurella multocida, Pasteurella hemolytica)* , contagious pleuropneumoniae (*Mycoplasma mycoides),* bovine mastitis (*Mycoplasma alkalescens*, *M*. *canadense*, *M*. *bovis*, *M*. *laidlawii) ,* paratuberculosis, tuberculosis, actinomicosis, actinobacilosis, leptospirosis, coccidioidomicosis, coccidiosis, erlichiosis.
- In equines:: Equine rinoneumonitis (α herpesvirus, type I and IV), equine exanthema coital (α herpesvirus, tipo-3), equine influenza (orthomyxovirus), parainfluenza-3 (paramyxovirus), equine infectious anemia (lentivirus), papilomatosis (papilomavirus), histoplasmosis, coccidiosis, erlichiosis, leptospirosis.
- In canine:: Canine distemper (morbillivirus), canine parainfluenza cSv-5 (paramyxovirus), herpesvirus (α herpesvirus), Aujesky's disease (α herpesvirus) papilomatosis (papilomavirus), histoplasmosis, coccidioidomicosis, criptococosis, blastomicosis, criptococosis, aspergilosis, candidacies, coccidiosis, toxoplasmosis, neosporosis, leishmaniasis, cryptosporidiosis, balantidiasis, amebiasis, erlichiosis, leptospirosis, brucellosis.
- In felines:: Feline rinotraqueitis (α herpesvirus), feline urolithiasis (β herpesvirus), Aujesky's disease (α herpesvirus), feline infectious peritonitis (coronavirus), feline viral leukemia (oncovirus).
- In pigs:: Herpesvirus of lactant pig (α herpesvirus), atrophic rhinitis (β herpesvirus, *Bordetella bronchiséptica, Hemophilus suis, Pasteurella multocida),* Aujesky's disease α (herpesvirus), porcine influenza (orthomyxovirus), transmissible gastroenteritis (coronavirus), hemagglutinin encefalomielitis (coronavirus), blue eye disease (paramyxovirus), enzootic pneumonia of pigs (*Mycoplasma hyopneumoniae) ,* porcine dysentery (*Treponema hyodisenteriae),* coccidiosis, balantidiasis, leptospirosis, tuberculosis.
- In ovines:: Pulmonary Adenomatosis (γ herpesvirus), Aujesky's disease (α herpesvirus), parainfluenza-3 (paramyxovirus), hemorrhagic bovine plague (morbillivirus), septicemia (*Pasteurella hemolytica, Pasteurella multocida)* , enteritis by coronavirus, campylobacteriosis, contagious ecthyma, paratuberculosis, tuberculosis, coccidiosis, mycoplasmosis, brucellosis, toxoplasmosis, erlichiosis.
- In birds:: Infectious Laryngotracheitis (α herpesvirus), Marek's Disease (γ herpesvirus), bird plague (orthomyxovirus), Newcastle's disease (paramyxovirus), infectious Bronchitis (coronavirus), bird leucosis (oncovirus), chronic respiratory disease (*Mycoplasma gallisepticum)* , infectious sinovitis (*Mycoplasma sinoviae)* , coccidiosis, bird cholera (*Pasteurella multocida)* , aspergillosis.

Also, DLE of present invention has characteristics of stability in shelf that allow it to conserve their therapeutic properties to 4°C during a period of 12 months, preferably of 7 to 8 months, by means of the application of ozone in amounts of 0.2 to 0.5 ppm, preferably to 0.3 ppm. Of amaze way, although ozone is a highly reactive substance with reducing properties, therapeutic properties of DLE obtained in this work were not modified, being increased solely conservation time.

On the other hand, methods of obtaining DLE of present invention allow obtaining high performances from lymphoid tissues, such as lymph nodes, spleen and thymus for example, either of previously sensitized animals to antigens related to diseases to be treated or no sensitized animals. These methods allow to obtain DLE with concentrations equivalent to 6 X 10⁶ to 1.6 X 10⁷ leukocytes / ml from blood, of 1 X 10⁷ to 8.3 X 10⁷ leukocytes / g from spleen, of 9 X 10 ⁶ to 6 X 10⁷ leukocytes / g from thymus and of 1.6 X 10⁷ to 1.8 X10⁹ leukocytes / g from lymph nodes.

On the other hand, methods of obtaining DLE of present invention allow to obtain a great amount and variety of individual doses of DLE to be administered in small volumes to the animal, with which, unnecessary annoyances to the patient by their administration are avoided. Also, this allows the administration to a greater number of patients and to fit the concentration of DLE with a greater facility to the necessary doses according during the course of the treatment and without obtaining considerable volumes. According to one of the modalities of the invention, the obtained volume of the doses of DLE to administer to the patient can fluctuate from 0.5 to 2 ml by dose, preferably from 1 to 1.7 ml and more preferably from 1.3 to 1.5 ml.

The obtained yields of DLE in this one work, allows obtaining individual doses for each scheme of treatment, with which therapeutic kits can be obtained containing DLE in the amounts and doses adapted for the treatment of the patients. In general, cases of 6 to 10 individual doses of DLE, preferably of 6 to 8 individual doses for the mentioned diseases can be obtained.

Like a modality of the therapeutic kit of the invention, the kit include initial individual doses to the treatment of 3 to 300 higher times in concentration (of 3 X 10⁶ to 5 X 10⁸) that the later proportionate doses of (1 X 10⁵ to 1.5 X 10⁶).

In a preferred modality of therapeutic kit, this one can be conformed by individual doses of DLE in concentrations equivalent to 3 X 10⁶ to 5 X 10⁸ lymphocytes. Such doses would conform from the 30 to 50% of the total of individual doses contained in the case, whereas the rest of the individual doses would have concentrations of DLE equivalent to 1 X 10⁵ to 1.5 X 10⁶ lymphocytes, conforming from the 50 to 70% of the total of contained individual doses in the case.

Also the source of obtaining DLE of the initial individual doses of the case, can preferably be of lymphatic, preferably lymphatic sanguineous tissue or and the more of spleen or thymus; in relation to the source of obtaining DLE of the final individual doses of the case it can be of sanguineous or lymphatic tissue, preferably sanguineous. Also, lymphatic or sanguineous tissues from where it obtains DLE can be derived from animals previously sensitized or immunized with the antigen of interest or without sensibilitation or previous immunization.

The medical use of the present invention includes a previous selection of the patients to receive the treatment, process in which important clinical signs of the disease are detected, such as positive feedbacks to laboratory tests, within which they are single to illustrate the invention, the tests of agglutination in plate, direct immunofluorescence in frotis of epithelia, direct seric micro agglutination, coproparasitoscopic examination, seroneutralization, bacterial isolation and anatomopathologic and microbiological diagnosis, among others.

The scheme of treatment developed in the present work includes the administration of DLE to patient in doses equivalent to 1.0 X 10⁵ leukocytes to 5 X 10⁸ leukocytes by Kg of weight, preferably to doses equivalent to 1.0 X 10⁶ leukocytes to 5 X 10⁸ leukocytes by Kg of weight or the more preferably to doses equivalent to 1.5 X 10⁶ leukocytes to 5 X 10⁶ leukocytes by Kg of weight. Such doses can be applied in unique doses or each 24 to 96 hours, preferably every 48 hours and until a period of 15 to 30 days or until the complete reestablishment of the patient. The administration of DLE is made via parenteral, and although the via subcutaneous is preferred; other tracts of administration can be used.

This scheme allows the administration of different doses from DLE with the purpose of making respond the patient of efficient way and maintain its recovery until the complete reestablishment of its state of health, for that reason, in a modality of the method of treatment of the present invention, greater doses of DLE in 50% of the total of individual doses administered during the treatment, preferably at the beginning of the same one. The rest of the individual doses would at the most have smaller concentrations in 70% of the total of administered doses. Preferably the percentage of individual doses with greater concentrations at the beginning of the treatment would be from the 30 to 50% of the total of administered individual doses, whereas for individual doses with smaller concentrations it would be from the 50 to 70% of the total of individual doses administered. Also the source of obtaining DLE in the initial phases of the treatment can be of lymphatic, preferably lymphatic sanguineous tissue or and the more preferably of spleen or thymus; in relation to the source of obtaining of DLE administered in the final phases and of maintenance of the treatment it can be of sanguineous or lymphatic tissue, preferably sanguineous.

Also, like an additional modality of the treatment of the present invention, leukocytes can be administered to individual doses of the same concentration equivalent to 1 X10⁵ to 5 X 10⁸ during the course of the treatment, preferably to doses equivalent to 1 X 10⁶ to 5 X 10⁸ leukocytes or the more preferably to doses equivalent to 1.5 X 10⁶ to 5 X 10⁶ leukocytes.

DLE of the present invention can be stored in packages designed for their therapeutic application, such as pharmaceutical glass containers that allows their conservation, or under conditions of refrigeration or freezing. The extracts can be obtained from liquid form or in solid form after putting them under a treatment by liofilization or another treatment that allows conserving its therapeutic qualities. If chosen distribution of DLE to the patient is by liofilizated, later DLE can be reconstituted using sterile injectable water addition or some other liquid therapeutically advisable for their administration.

For the aims of the present invention, DLE can be obtained through the following methodology:

### Obtaining dialyzed leulcocyte extract (DLE).

The elaboration of DLE requires diverse steps and each one can be carried out with different techniques from which the following ones are mentioned:

### 1. Selection and preparation of the donor.

DLE can be elaborated from tissues of an animal of the same species (homologous) or of another species different from the one from the receptor (heterologous), reason why the individual donor of lymphocytes can be of any species. If the objective is to obtain specific DLE for some antigen, it requires itself of the antigenic sensibilitation of the previous donor to the obtaining of lymphocytes. The sensibilitation can be made repeatedly inoculating the pathogenic microorganism via intranasal, intraperitoneal, intramuscular, intraskin, and subcutaneous among others and obtaining the sample of leukocytes when a mounted secondary immune response has adapted. In case of lacking a pathogenic microorganism, it is possible to be replaced by serial applications of vaccine, bacteria or commercial toxins administered by the same tracts. Also the specific antigenic sensitization by immunological tests can be determined, as *in vitro* as *in alive* that demonstrate the natural exhibition to the specific antigen. If the objective is to make unspecific DLE, it is not necessary to make the previous sensibilitation of animals.

### 2. Cellular harvesting.

The harvesting of leukocytes will be able to be carried out from:
a) Blood, obtaining it for example, by cardiac puncture, of the artery carotid, femoral, volume or of the retro-orbitarie sine, radial, cephalic, safena, jugular veins, or any other vessel that could be used for this aim,
b) Lymphoid Organs primary or secondary, such as thymus, spleen or lymph nodes.

In this one sense, and like a result of the present invention, the use of lymphoid organs allows the obtaining of significantly greater yields to the obtained by the well-known methods using like source of obtaining sanguineous tissue. However, for the aims of this one work, anyone of the mentioned tissues can be used for the obtaining of DLE with therapeutic activity.

The harvesting of blood can be made in bags for sanguineous transfusion or sterile bottles using heparin, acid citrus, dextrose, sodium citrate or EDTA like anticoagulant. The harvesting of organs will be able to be made using sterile bags or bottles, they will cool off of immediate way and later they will be disintegrated using macerated and sifted of the organs.

### 3. Obtaining of leukocytes and lymphocytes.

The separation of leukocytes or lymphocytes is made using separating agents by gradient of concentration like Ficoll hypacke, by manual separation with pipettes after centrifuging the samples, allowing the sedimentation of the sanguineous cellular package with the use or not of enhancers of sedimentation, such as fibrinogen and others, or through the use of the French press or some other means or well-known methodology for aims of cellular separation.

### 4. Cellular lysis.

The leukocyte lysis can be made using methods of cellular rupture like ultrasound, microwaves, chemical agents, differentials of osmotic pressure with distilled water or using methods of lysis by change of temperature and for this aim the freezing of leukocytes concentrate with liquefy nitrogen can be used, refrigeration to diverse temperatures (-20°C or -70°C), conventional freezing to 4°C, the mixture of dry-acetone ice and the later heating of the sample in bain Marie to 37°C, repeating the procedure the times that are necessary. In the entire cases one verifies that the lysis has been completed through the microscopic observation of the tissue.

### 5. Molecule separation.

Separation of molecules from leukocytes lysates can be carried out through membranes of dialysis with the capacity to retain bigger molecules from 10,000 to 12,000 Daltons or using the ultra filtration with peristaltic pump and membranes with smaller micro pores from 10,000 to 12,000 Daltons. The dialysis is made with distilled water either or with some conventional buffer.

The used vehicle to suspend molecules obtained in the process of separation using dialysis can be distilled or injectable water and for the ultra filtration process physiological saline solution, buffered saline solution or Hartman solution.

### 6. Sterilization.

The leukocyte extract obtained is sterilized by bubbling with ozone to 0,3 ppm.

### 7. Conservation.

The extract can conserve in refrigeration or freezing to inferior temperatures to 4°C, in liquid form or after making a liofilization process. Some antibiotic combination can be added of ample specter for the same aim. Also, it will be wrap in glass bottles properly sealed for his storage.

The following specific examples illustrated the application of the invention that allows using DLE for the treatment of diverse animal diseases. One will be due to consider that other examples could be applied for the infectious diseases more common of some animal species by some person with average knowledge in the issue, reason why the present invention is not limited to the following examples.

### Example 1. Obtaining DLE from sanguineous tissue.

Blood from the selected donating animal by means of puncture with syringe of femoral artery radial, cephalic, safena or jugular vein was obtained, according to the conditions of the donor. Blood was collected using commercial bags of transfusion for human use connected to the syringe using hoses, with capacity of 500 ml containing sodium citrate as anticoagulant. During the harvesting of blood, bag stayed in constant slight agitation and below the zone of puncture. Once contained blood within bag, a sample was taken and a leukocyte count using microscopic observation by duplicate was made, using pipettes of Thomas, liquid of Turk, and camera of Newbauer. Subsequent to the count, blood was put under centrifugation at 1,200 rpm by 20 minutes using a centrifuge with containers with a capacity of 500 ml.

Once finished the centrifugation, bag of container was retired and it was placed in a surface previously sterilized with chlorine and ultraviolet light, holding it with surgical clamps by the 2 superior ends to way that bag was hanging to later make a cut in the antero-superior part. The final stage of the liquid contained in bag was separated of the cellular package with glass pipettes and it was deposited in a glass bottle with hermetic cover congealing finally. Leukocytes thus obtained were lysated by freezing to -20°C using a commercial freezer and immediate heating to 38°C in bain Marie. The freezing-defrost process was made 10 times and the existence of lysis in 98% of the cells using count through camera of Newbauer using microscopic observation was verified. The obtained mixture was dialyzed with injectable water keeping a relation between the volume from this one and the volume contained in bag of 2:1 dialysis respectively. The dialysis was made during 48 hours to 4°C using membranes of dialysis with capacity of molecule retention greater to 12,000 Daltons (Sigma 50, diameter 49 by mm), previously washed and sterilized, placed in a glass bottle. Finished the dialysis process, the membrane was retired and the liquid contained in the glass bottle was sterilized using bubbling with 0.3 ppm of ozone during 5 to 10 min. Later the resulting liquid was distributed manually with plastic syringes in glass packages with capacity of 3 ml. Once made the filling of 10 bottles, sterile rubber covers were placed and they were sealed with aluminum gargoyles. The bottles with DLE were stored in refrigeration or freezing until their use. Using this method, it was obtained from 6,000 to 16,000 leukocytes by µl of blood Also, in relation to the number of dose to an equivalent concentration of 1.5 X 10⁶ leukocytes, obtained 10 doses by each 13,6 ml of blood.

### Example 2. Obtaining DLE from lymphatic tissue.

The lymphatic organ of interest (spleen, thymus, lymph nodes) of healthy individuals was collected under anesthetic euthanasia, by means of surgical extraction; once separated of tissue of surrounding organs and cleaned with distilled water, they stayed in freezing (-17°C) until its processing. The organ was placed in a bottle previously sterilized and they were added from 50 to 250 ml of physiological saline solution. Later a sample was taken from approximately 1 gr. of weight, which was macerated with 1 ml of physiological saline solution using a metallic micro grid, taking 5 µl with a Thomas's pipette and being diluted with Turk's liquid. The sample was placed in a Newbauer camera and a cellular count using microscopy was made. At the same time, the organ was cut in small pieces with surgical scissors and macerated with a mixer previously sterilized using 50 to 200 ml of physiological saline solution. The resulting macerate was placed in glass bottles with hermetic cover and was congealed.

Leukocytes thus obtained were lysated by freezing to -20°C using a commercial freezer and immediate heating to 38°C in bain Marie. The freezing-defrost process was made 10 times and the existence of lysis in 98% of the cells was verified using Newbauer camera to count and using microscopic observation. The obtained mixture was dialyzed with injectable water keeping a relation between the volume from this one and the volume contained in dialysis of 2:1 respectively. The dialysis was made during 48 hours to 4°C using membranes of dialysis with capacity of molecule retention greater to 12,000 Daltons (Sigma 50, diameter 49 by mm), previously washed and sterilized, placed in a glass bottle. Finished the dialysis process, the membrane was retired and the liquid contained in the glass bottle was sterilized via bubbling with 0.3 ppm of ozone for the period of 5 to 10 min. Later the resulting liquid was distributed manually with plastic syringes in glass packages with capacity of 3 ml. Once made the filling of 10 bottles, sterile rubber covers were placed and they were sealed with aluminum gargoyles. The bottles with DLE were stored in freezing until their use.

Using this one method it was obtained from 10,000 to 70,000 leukocytes by µg of tissue in the case of spleen, of 9,000 to 60,000 leukocytes by µg of tissue in the case of thymus and 16,000 to 230,000 leukocytes by µg of tissue in the case of lymph nodes. Also, in relation to the number of dose to an equivalent concentration of 1.5 X 10⁶ leukocytes, obtained 10 doses by each 1.8 gr. of spleen, 10 doses by each 4.4 gr. of thymus and 10 doses by each 0.08 gr. of lymph nodes.

DLE was obtained from sensitized animals to the disease treated in the corresponding clinical analysis, previous to the sacrifice, immunized with a dose of vaccine containing the antigen of interest with particular schemes of administration as required in the analysis.

### Example 3. Obtaining DLE from canine blood.

Adult Rottweiler dogs were used like blood donors, clinically healthy, with its complete and effective calendars of vaccination (antirabic, triple and parvovirus vaccine), wormed annually and with an adapted nutritional state. From donors 450 ml were obtained of complete blood by means of bleeding and with the purpose of obtaining DLE, DLE was processed according to specified in example no. 1, DLE was obtained from sensitized animals to the disease treated in the corresponding clinical analysis, 15 days before bleeding a dose of vaccine containing the interest antigen was applied. The sanguineous donations were made every 60 days until finalizing the corresponding clinical analysis.

### Example 4. Obtaining DLE from bovine blood.

Blood of clinically healthy bull Holstein race was used of a year six months, vaccinated exclusively against brucellosis and without no antecedent of vaccination against bovine viral Rinotraqueitis, parainfluenza, leptospirosis, pasteurelosis, symptomatic carbon nor malignant edema. From donors 450 ml were obtained of complete blood by means of bleeding and with the purpose of obtaining DLE, it was processed according to the specified in example no. 1. DLE was obtained from sensitized animals to the disease treated in the corresponding clinical analysis, it was applied via subcutaneous a double dose of canine triple vaccine (canine distemper, hepatitis and Leptospira) 30, 20 and 10 days previous to the sanguineous harvesting.

### Example 5. Obtaining DLE from pig blood.

Blood of no sensitized pig procreate in a commercial farm was used. From donors 450 ml were obtained of complete blood by means of bleeding and with the purpose of obtaining DLE, it was processed according to the specified in example no. 1. DLE was obtained from sensitized animals to the disease treated in the corresponding clinical analysis; they immunized with dose of vaccine containing the antigen of interest with particular schemes of administration as required in the analysis.

### Example 6. Obtaining DLE from equine blood.

Blood of adult creoles horse, clinically healthy, not sensitized originating of Rastro market was used. From the donors 15 liters of complete blood by means of bleeding were obtained and with the purpose of obtaining DLE, it was processed according to the specified in example no. 1. In case of DLE were obtained from animals sensitized to the disease treated in the corresponding clinical analysis, immunized with dose of vaccine containing the antigen of interest with particular schemes of administration as required in the analysis.

### Example 7. Standardization DLE dose.

Previous to the lysis of leukocytes, the number of these, obtained of the separation process, was entered by the use of Newbawer's camera. After obtained the account of leukocytes of the 4 quadrants of the camera, the obtained amounts were added and they were multiplied by 50 to obtain the total number of leukocytes by µl contained in the fluid. After made the cellular lysis, to obtain the suitable dose to be applied in the analyses, this one calculated with respect to the total no. of cells before the lysis and later dilutions adapted with sterile distilled water until their obtaining were made. The obtained doses adjusted to a total volume of 1.5 ml, dose adapted for an animal of 10 Kg of weight. For the aims of the present invention, to describe the obtained doses as it were described in this one example, they will be denominated like "equivalent dose (number) of leukocytes".

### Example 8. Selection of canine patients for treatment of disease.

So that a patient could be considered as candidate to enter to the clinical analysis had to fulfill the following criteria:

### For the case of canine distemper:

1. Of inclusion: to present indicative clinical signs of infection of the disease, not to present neurological signs before initiating treatment and to demonstrate the viral infection by means of the test of direct immunofluorescence in frotis of conjunctival epithelium, sanguineous or vesical
2. Of exclusion: not to have proprietor, to suffer severe undernourishment, to suffer diseases or concurrent pathologies.
3. Of elimination: suspension of the treatment, death by another cause different from the infection by canine distemper, deviation of the patient, not to have the patient under ceiling and shelter, do not provide the prescribed diet

### For the case of leptospirosis:

1. Of inclusion: to present indicative clinical signs of infection by *Leptospira interrogans* to demonstrate the infection by means of the test of direct serum micro agglutination with titles up to 1/100.
2. Of exclusion: not to have proprietor, to suffer severe undernourishment, to suffer moderate to severe dehydration, to have signs of uremia, to present levels of creatinine superior to 2 mg/dl, to suffer diseases or concurrent pathologies.
3. Of elimination: suspensions of the treatment, death during the development of the clinical analysis, do not provide the prescribed diet.

### Example 9. Previous preparation to treatment.

The patients including in the analyses received antimicrobial therapy, previous to DLE administration. The therapy consisted of the administration of amoxicillin (11 mg/Kg each 12 hrs. via oral) and gentamicin (4 mg/Kg each 24 hrs. via intramuscular) by 7 days. Each patient was evaluated of individual way and the therapeutic procedures for their necessary clinical stabilization were applied. For each one of patients including in the analyses, the power necessities were calculated (with base in formula [corporal weight X 30 + 70 ] x 2), which were administrated with pelletized dry commercial food. Patient was under ceiling and shelter 24 hrs day while treatment last, allowing momentary exits for the excretion of decals dejections and urine.

### Example 10. Evaluation of results.

The criteria of success discrimination either failure of the treatment was of alive or dead or of negative or positive or to the test of agglutination in plate, according to the corresponding analysis. The obtained results, unless the opposite indicates itself, were put under a statistical evaluation by means of the test of Ji-square.

### Example 11. Treatment of canine distemper with DLE of blood of sensitized dog.

This example corresponds to a prospective, longitudinal and observational clinical analysis where obtained DLE by blood of sensitized dog to canine distemper (as it is in example no. 3) a dose equivalent to 1.5 X 10⁶ leukocytes by Kg of weight was used with intervals of 48 hrs via subcutaneous for the treatment of canine distemper. For this analysis 370 dogs naturally infected with the canine virus of canine distemper were used and they were included alternatively in group A or in group B, 185 animals were integrated in group A and the other 185 dogs formed group B. Dogs were selected according to the described in example no. 8 and it did not consider race, age, sex, nor previous vaccinations. Also, the patients were treated previous to the treatment with DLE according to the indicated in example no. 9.

After obtained the extract, this one standardized to a concentration equivalent to 1.5 X 10⁶ leukocytes by dose, according to as it is in example no. 7.

To dogs including in group A was applied DLE via subcutaneous to an equivalent dose to 1, 5 X 10⁶ leukocytes to them by Kg of weight with an interval between 48 doses of during 10 days, followed of intervals between each 96 doses of hrs. until the patient was clinically healthy for 15 days or until his death. To animals of group B physiological saline solution was applied to them, with the same frequency of application that in group A. Group A, had a population route to a month to eight years (average of two years five months), being 60.5 % of the smaller population of a year of age. Group B, presented a population route of two months to seven years, (average of two years a month), being 65.7 % of the smaller population of a year of age. Results obtained, after the treatment, are in the following table:

| **Group** | **DLE dose*** | **Survival**** | **Mortality**** |
|---|---|---|---|
| A | 1.5 X 10⁶ | 90 | 10 |
| B | - | 13 | 87 |

| | | | |
|---|---|---|---|
| * By Kg of weight ** Expressed in percentage | | | |

After the evaluation of the results as it is indicated in example no. 10, under the design of this one clinical analysis the application of DLE in the canine treatment of canine distemper remarkably increases the survival of dogs affected at highly significant levels (p ≤ 0,01).

### Example 12. Canine treatment of canine distemper with different DLE doses of blood of sensitized dog.

In this example one describes to the use of the obtained DLE of blood of dog sensitized (as one is in example no. 3) to dose equivalent to 5 X 10⁸ leukocytes in unique dose, 5 X 10⁶ leukocytes by Kg of corporal weight each 48 hrs. by 10 days, 1.5 X 10⁶ leukocytes by Kg of corporal weight each 48 hrs. by 10 days and 1 x 10⁵ leukocytes by Kg by 10 days, by via subcutaneous, in ill dogs of canine distemper. In this analysis 50 dogs of three months to a year of age were used, infected naturally with the canine virus of canine distemper, which were numbered and included progressively in groups A, B, C, D or and according to the moment at which they appeared for his attention, with which five groups of 10 dogs integrated themselves each one. The patients were selected according to the described in example no. 8 and she did not consider race, age, sex, nor previous vaccinations. Also, the patients were treated previous to the treatment with DLE according to the indicated in example no. 9.

To dogs including in group A was applied a DLE dose equivalent to 5 X 10⁸ leukocytes to them in unique application. To animals of group B was applied to a dose equivalent to 5 X10⁸ leukocytes by Kg of corporal weight each 48 hrs. by 10 days To group C a DLE dose equivalent to 1.5 X 10⁶ leukocytes by Kg of corporal weight was applied each 48 hrs. by 10 days. For dogs of group D a dose equivalent to 1 x 10⁵ leukocytes was used each 48 Kg hrs. by 10 days. To dogs of group E and physiological saline solution was applied to them. In all patients via subcutaneous was used. Results obtained, after the treatment, are in the following table:

| **Group** | **DLE dose*** | **Survival**** | **Mortality**** |
|---|---|---|---|
| A | 5 x 10⁸ | 80 | 20 |
| B | 5 x 10⁶ | 100 | 0 |
| C | 1.5 x 10⁶ | 100 | 0 |
| D | 1 x 10⁵ | 50 | 50 |
| E | - | 0 | 100 |

| | | | |
|---|---|---|---|
| * By Kg of weight. ** Expressed in percentage. | | | |

After the evaluation of the results as it is indicated in example no. 10, under the design of this clinical analysis the application of DLE in canine treatment of canine distemper of treated dogs with DLE doses of groups A, B, C statistically increases the survival of dogs affected at highly significant levels (p ≤ 0,01) when comparing them with group control (group E). When analyzing the obtained results of the treatments of dogs in groups A, B and C statistically significant difference was not observed (p ≥0,05), nevertheless between the treatments of dogs in groups B and C the treatments were superior with significant differences (p ≤0,05) when comparing it with group D.

### Example 13. Treatment of canine distemper with DLE of blood of sensitized dog, lymph nodes and spleen.

This example describes the use of DLE obtained from blood (as it is in example no. 3), lymph nodes and spleen of dog for the canine treatment of canine distemper. In this case 45 dogs of four months to of a year of age were used, infected naturally with the canine virus of canine distemper, which were numbered and they were included progressively in groups A, B and C according to the moment at which they appeared for his attention, with which three groups of 15 dogs integrated themselves each one. Dogs were selected according to the described in example no. 8 and it did not consider race, age, sex, nor previous vaccinations. Also, the patients were treated previous to the treatment with DLE according to the indicated in example no. 9.

For the elaboration of DLE from tissue, lymph nodes and spleens of sacrificed dogs were used different from the donors of the lymph nodes as it is described in example no. 2. After obtained the extract, this one standardized to a concentration equivalent to 1.5 X 10⁶ leukocytes by dose, according to as it is in example no. 7.

To dogs including in group A was applied a DLE dose of sanguineous origin equivalent to 1.5 X 10⁶ leukocytes by Kg of corporal weight each 72 hrs by 10 days followed of applications by other 15 days each 48 hrs. To group B it was applied to DLE of lymph nodes to the same dose and frequency of application. For dogs of group C DLE prepared of spleen to the same dose of leukocytes and the same frequency was used to dogs of groups A and B. In all patients application was via subcutaneous. Results obtained, after the treatment, are in the following table:

| **Group** | **Source of DLE*** | **Survival**** | **Mortality**** |
|---|---|---|---|
| A | Blood | 93.3 | 6.7 |
| B | Lymph nodes | 86.7 | 13.3 |
| C | Spleen | 93.3 | 6.7 |

| | | | |
|---|---|---|---|
| * Canine. ** Expressed in percentage. | | | |

After the evaluation of results as it is indicated in example no. 10, under the design of this one clinical analysis, can affirm that there is statistically no significant difference between the three training groups (p >0,01) when tissue using DLE different (blood, lymph nodes and spleen).

### Example 14. Treatment of canine distemper with DLE of sensitized and not sensitized bovine blood.

This example corresponds to a prospective, cross-sectional, observational clinical analysis that describes the use of DLE obtained from blood of sensitized bovines, with canine triple vaccine, not sensitized. Applied to a dose equivalent to 1.5 X 10⁶ leukocytes by Kg of weight with intervals of application of each 48 hrs. via subcutaneous for the treatment of canine distemper. In this case 45 dogs of four months to a year of age were used, infected naturally with the canine virus of canine distemper, which were numbered and they were included progressively in groups A, B and C according to the moment at which they appeared for his attention, three groups of 15 dogs were integrated. Dogs were selected according to the described in example no. 8 and it did not consider race, age, sex, nor previous vaccinations. Also, patients were treated previous to the treatment with DLE according to the indicated in example no. 9.

The lymphocyte extract (DLE) of bovine was obtained according to as described in example no. 4, whereas DLE of dog was obtained according to as in example no. 3. After obtained the extracts, these standardized to a concentration equivalent to 1.5 X 10⁶ leukocytes by dose, according to as in example no. 7.

To dogs including in group A was applied via subcutaneous a DLE dose of sensitized bovine origin equivalent to 1.5 X 10⁶ leukocytes by Kg of corporal weight each 48 hrs. by 10 days and each 72 hrs. by 15 days. To group B a DLE dose of not sensitized bovine origin was applied by the same via, and frequency of application. And to group C was applied DLE of canine origin of identical form that in the other two groups.

The results obtained, after the treatment, are in the following table:

| **Group** | **Resource of DLE*** | **Survival**** | **Mortality**** |
|---|---|---|---|
| A | sensitized Bovine | 100 | 0 |
| B | no sensitized Bovine | 93.3 | 6.7 |
| C | no sensitized Canine | 93.3 | 6.7 |

| | | | |
|---|---|---|---|
| * Blood. ** Expressed in percentage. | | | |

After the evaluation of the results as it is indicated in example no. 10, under the design of this one clinical analysis it can affirm that there is no statistical difference between the three training groups (p >0,01) when sensitized bovine using DLE, bovine not sensitized or sensitized dog not to treat canine distemper.

### Example 15. Treatment of canine distemper with DLE from lymph nodes and spleen of sensitized bovine.

This example corresponds to a prospective, cross-sectional and observational clinical assay, that describes the use of DLE obtained from lymph nodes and spleen of sensitized bovines to a dose equivalent to 1.5 X 10⁸ leukocytes by Kg of weight with intervals of each application of 48 hrs via subcutaneous for the treatment of canine distemper. In this assay, 45 dogs of four months to a year of age were used, infected naturally with the canine virus of canine distemper, which were numbered and they were included progressively in groups A, B and C according to the moment at which they appeared for his attention. With this strategy three groups of 15 dogs each one were integrated. Dogs were selected according to the described in example no. 8 and it did not consider race, age, sex, nor previous vaccinations. Also, patients were treated before treatment with DLE according to the indicated in example no. 9.

For the elaboration of DLE from tissue, lymph nodes and spleens of obtained adult Creole bovines of a municipal sign were used, which once cleaned were maintained in freezing (-17°C) until their processing, according to example no. 2. It is possible to mention that age was not known, sex, previous vaccinations and type of feeding of the donating animals. DLE from dog blood was obtained according to example no. 3. After obtained the extracts, these were standardized to a concentration equivalent to 1.5 X 10⁶ leukocytes by dose, according to example no. 7.

To dogs including in group A, DLE from bovine splenic origin not sensitized was applied to them to a dose equivalent to 1.5 X 10⁶ leukocytes by Kg of corporal weight via subcutaneous each 48 hrs. by 10 days and each 72 hrs. by 15 days. To group B, DLE from lymph node of bovine not sensitized origin was applied to them by the same way, dose and frequency of application. To group C, DLE of canine hematic origin was applied on identical form to them that the other two groups.

Results obtained, after the treatment, are in the following table:

| **Group** | **Source of DLE⁺** | **Survival**** | **Mortality**** |
|---|---|---|---|
| A | Spleen | 86.6 | 13.3 |
| B | Lymph nodes | 93.3 | 6.7 |
| C | Blood* | 100 | 0 |

| | | | |
|---|---|---|---|
| + sensitized Bovine ** Expressed in percentage * sensitized Canine. | | | |

After evaluation of results as indicated in example no. 10, under the design of this clinical assay it is possible to be affirmed that there is no significant statistical difference between the three groups under study (p >0.05) when using DLE from spleen or lymph node of bovine sensitized, when comparing it with DLE elaborated with blood of sensitized dog to treat canine distemper.

### Example 16. Treatment of canine distemper with DLE from blood, lymph nodes and spleen of sensitized pig.

This example corresponds to a prospective, cross-sectional and observational clinical assay that describes the use of DLE obtained from blood, lymph nodes and spleen of sensitized pigs to a dose equivalent to 1.5 X 10⁶ leukocytes by Kg of weight with intervals of each application of 48 hrs via subcutaneous for the treatment of canine distemper. In this assay 40 dogs to four months to a year of age were used, infected naturally with the canine virus of canine distemper, which were numbered and they were included progressively in groups A, B and C according to the moment at which they appeared for his attention. With this strategy four groups of 10 dogs each one were integrated. Dogs were selected according to the described in example no. 8 and it did not consider race, age, sex, nor previous vaccinations. Also, patients were treated previous to the treatment with DLE according to the indicated in example no. 9.

For the elaboration of DLE lymph nodes were used, spleens and blood from 3 bred pigs in a commercial farm, which were immunized twice with double doses of canine triple vaccine (canine distemper, hepatitis, Leptospira) with intervals between dose of 10 and 15 days previous to the sacrifice. The extracts were obtained according to example no. 2. DLE from dog blood was obtained according to example no. 3. After obtained the extracts, these were standardized to a concentration equivalent to 1.5 X 10⁶ leukocytes by dose, according to example no. 7.

To dogs including in group A, DLE from splenic origin sensitized pig was applied to them to a dose equivalent to 1.5 X 10⁶ leukocytes by Kg of corporal weight via subcutaneous each 48 hrs. by 10 days and each 72 hrs. by 15 days. To group B, DLE from lymph node of sensitized pig was applied to them by the same one way, dose and frequency of application. To group C, DLE of porcine hematic origin was applied on identical form to them that the other two groups. Finally it was injected DLE of sanguineous origin from sensitized canine by the same via and the same dose to dogs of group D. Results obtained, after the treatment, are in the following table:

| **Group** | **Source of DLE*** | **Survival**** | **Mortality**** |
|---|---|---|---|
| A | Spleen | 80 | 20 |
| B | Lymph nodes | 90 | 10 |
| C | Blood | 90 | 10 |
| D | Blood⁺ | 100 | 0 |

| | | | |
|---|---|---|---|
| * Sensitized pig. +Sensitized Canine ** Expressed in percentage. | | | |

After evaluation of results as indicated in example no. 10, under the design of this clinical assay it is possible to be affirmed that there is no significant statistical difference between the four groups under study (p >0.05) when using DLE from blood, spleen or lymph node of sensitized pigs and DLE elaborated with blood of sensitized dog to treat canine distemper.

### Example 17. Treatment of canine distemper with DLE from blood, lymph nodes and spleen of horse no sensitized.

This example corresponds to a prospective, cross-sectional and observational clinical assay that describes the use of DLE obtained from blood, lymph nodes and spleen of no sensitized horses to a dose equivalent 1.5 X 10⁶ leukocytes by Kg of weight with intervals of each application of 48 hrs via subcutaneous for the treatment of canine distemper. In this assay 40 dogs to four months to a year of age were used, infected naturally with the canine virus of canine distemper, which were numbered and they were included progressively in groups A, B, C and D according to the moment at which they appeared for his attention. With this strategy four groups of 10 dogs each one were integrated. Dogs were selected according to the described in example no. 8 and it did not consider race, age, sex, nor previous vaccinations. Also, patients were treated before the treatment with DLE according to the indicated in example no. 9.

For the elaboration of DLE lymph nodes were used, spleens and blood from sacrificed equines in a municipal sign. Extracts were obtained according to example no. 2. DLE from dog blood was obtained according to example no. 3. After obtained the extracts, these were standardized to a concentration equivalent to 1.5 X 10⁶ leukocytes by dose, according to example no. 7.

To dogs including in group A, DLE from splenic of not sensitized equine origin was applied to them to a dose equivalent to 1.5 X 10⁶ leukocytes by Kg of corporal weight via subcutaneous each 48 hrs. by 10 days and each 72 hrs. by 15 days. To group B, DLE from lymph node of not sensitized equine was applied to them by the same one way, dose and frequency of application. To group C, DLE of equine hematic origin was applied on identical form to them that the other two groups. Finally to group D, DLE of canine blood was applied to them with the same application criteria that the previous groups. Results obtained, after the treatment, are in the following table:

| **Group** | **Source of DLE*** | **Survival**** | **Mortality**** |
|---|---|---|---|
| A | Spleen | 90 | 10 |
| B | Lymph nodes | 90 | 10 |
| C | Blood | 100 | 0 |
| D | Blood⁺ | 90 | 10 |

| | | | |
|---|---|---|---|
| * No sensitized Equine ** Expressed in percentage +Sensitized Canine | | | |

After evaluation of results as indicated in example no. 10, under the design of this clinical assay it is possible to be affirmed that there is no significant statistical difference between the three training groups (p 0.05) when using DLE from hematic, splenic or lymphatic origin of no sensitized equine and DLE elaborated with blood of sensitized dog to treat canine distemper.

### Example 18. Treatment of canine leptospirosis with DLE of spleen of sensitized dog.

This example corresponds to a prospective, cross-sectional and observational clinical assay that describes the use of DLE obtained from spleen of sensitized dogs to a dose equivalent to 1.5 X 10⁶ leukocytes by Kg of weight with intervals of each application of 48 hrs via subcutaneous for the treatment of canine leptospirosis. In this assay 30 dogs, naturally infected with *Leptospira interrogans* were used, which numbered them by appearance order and they were included alternatively in to groups A and B according to the moment at which they appeared for his attention. With this disposition two groups of 15 dogs each one were integrated. Dogs were selected according to the described in example no. 8 and it did not consider race, age, sex, nor previous vaccinations. For each one of patients including in the assays, the energetic necessities were calculated (with base on formula [corporal weight X 30 + 70] x 2), which were contributed with dry pelletized commercial food. The dog was maintained under ceiling and shelter 24 hrs at day while treatment was running, allowing single momentary exits for the excretion of fecal and urine dejections.

For the elaboration of DLE from tissue, spleens of sacrificed dogs were used as described in example no. 2. After obtained the extracts, these were standardized to a concentration equivalent to 1.5 X 10⁶ leukocytes by dose, according to example no. 7. To dogs including in group A, were applied to them to a dose equivalent to 1.5 X 10⁶ leukocytes by Kg of corporal weight via subcutaneous each 48 hrs. by 15 days. To group B it was applied procainic penicillin G (40,000 U/Kg) and streptomycin (22mg/Kg) each 24 hrs. by 15 days.

The clinical signs were described from the beginning of the assay and every 7 days, by the following way:
**0** when there are no signs.
**1** when the patient presented single prodromics signs.
**2** when the specific signs were limited to vomit, diarrhea, poliury, hematury.
**3** when ictericia was observed, hematury, diarrhea with melena, hematemesis.

Results obtained, after the treatment, are in the following tables:

| **Clinical valuation group A** | | | | |
|---|---|---|---|---|
| **Dog** | **Initial evaluation** | **Day 7** | **Day 14** | **Day 21** |
| 1 | 3 | 3 | 2 | 1 |
| 2 | 2 | 2 | 1 | 0 |
| 3 | 2 | 2 | 0 | 0 |
| 4 | 3 | 2 | 1 | 0 |

| **Clinical valuation group A** | | | | |
|---|---|---|---|---|
| **Dog** | **Initial evaluation** | **Day 7** | **Day 14** | **Day 21** |
| 5 | 3 | 1 | 0 | 0 |
| 6 | 2 | 1 | 0 | 0 |
| 7 | 3 | 2 | 1 | 0 |
| 8 | 3 | 2 | 0 | 0 |
| 9 | 2 | 1 | 0 | 0 |
| 10 | 2 | 0 | 0 | 0 |
| 11 | 2 | 2 | 1 | 1 |
| 12 | 3 | 1 | 0 | 0 |
| 13 | 2 | 1 | 1 | 0 |
| 14 | 2 | 1 | 0 | 0 |
| 15 | 2 | 0 | 0 | 0 |

| **Clinical valuation group B** | | | | |
|---|---|---|---|---|
| **Dog** | **Initial evaluation** | **Day 7** | **Day 14** | **Day 21** |
| 1 | 2 | 1 | 0 | 0 |
| 2 | 2 | 2 | 1 | 0 |
| 3 | 3 | 2 | 0 | 0 |
| 4 | 3 | 2 | 1 | 1 |
| 5 | 2 | 0 | 0 | 0 |
| 6 | 2 | 1 | 1 | 0 |
| 7 | 3 | 2 | 1 | 0 |
| 8 | 2 | 2 | 0 | 0 |
| 9 | 3 | 1 | 1 | 0 |
| 10 | 3 | 2 | 2 | 0 |
| 11 | 3 | 2 | 0 | 0 |
| 12 | 2 | 1 | 0 | 0 |
| 13 | 2 | 1 | 1 | 1 |
| 14 | 3 | 1 | 0 | 0 |
| 15 | 3 | 2 | 1 | 1 |

Also, for these groups it was made a serologic analysis at the beginning of the treatment as well as another 21 days later to detect antibodies anti-*Leptospira* by the micro agglutination technique with alive antigen.

Results of this test are next:

| **Titers of anti-Leptospira antibodies. Group A** | | | |
|---|---|---|---|
| **Dog** | **Initial titer** | **21 Days titer** | **Sero-variety** |
| 1 | 1/3200 | 1/50 | *L pomona* |

| **Titers of anti-Leptospira antibodies. Group A** | | | |
|---|---|---|---|
| **Dog** | **Initial titer** | **21 Days titer** | **Sero-variety** |
| 2 | 1/400 | - | *L. pomona* |
| 3 | 1/800 | - | *L. grippotyphosa* |
| 4 | 1/1600 | - | *L. pyrogenes* |
| 5 | 1/400 | - | *L. canicola* |
| 6 | 1/200 | 1/50 | *L. wolffi* |
| 7 | 1/800 | 1/100 | *L. icterohemorragiae* |
| 8 | 1/3200 | 1/800 | *L. grippotyphosa* |
| 9 | 1/400 | 1/100 | *L.autumnalis* |
| 10 | 1/100 | - | *L. canicola* |
| 11 | 1/100 | - | *L. pomona* |
| 12 | 1/400 | - | *L. tarassovi* |
| 13 | 1/200 | - | *L. hardjo* |
| 14 | 1/100 | - | *L. icterohemorragiae* |
| 15 | 1/100 | - | *L. pomona* |

| **Titers of anti-Leptospira antibodies. Group B** | | | |
|---|---|---|---|
| **Dog** | **Initial titer** | **21 Days titer** | **Sere-variety** |
| 1 | 1/400 | - | *L. pyrogenes* |
| 2 | 1/200 | - | *L. canicola* |
| 3 | 1/1600 | 1/100 | *L. canicola* |
| 4 | 1/3200 | 1/200 | *L. icterohemorragiae* |
| 5 | 1/100 | - | *L. grippotyphosa* |
| 6 | 1/100 | - | *L. pomona* |
| 7 | 1/800 | 1/50 | *L. pomona* |
| 8 | 1/400 | 1/50 | *L. tarassovi* |
| 9 | 1/800 | - | *L. wolffi* |
| 10 | 1/1600 | 1/100 | *L.wolffi* |
| 11 | 1/1600 | 1/50 | *L. pyrogenes* |
| 12 | 1/200 | - | *L. canicola* |
| 13 | 1/200 | - | *L. pomona* |
| 14 | 1/800 | - | *L. ballum* |
| 15 | 1/400 | - | *L. hardjo* |

The obtained results were set under a statistical evaluation. In the case of the valuation of the clinical signs the test of Kruskall-Wallis was used and for the case of the micro agglutination the t of Student test. After evaluation of results under the design of this clinical assay it is possible to be affirmed that there is no significant statistical difference between groups under study (p >0.05) when using DLE from not sensitized canine origin and the treatment with antimicrobial compounds. In other words both treatments were equally effective against the canine leptospirosis.

### Example 19. Treatment of canine brucellosis with DLE from spleen of no sensitized dog.

This example corresponds to a prospective, cross-sectional and observational clinical assay that describes the use of DLE obtained from spleen of no sensitized dogs to a dose equivalent to 1.5 X 10⁶ leukocytes by Kg of weight with intervals of each application of 48 hrs via subcutaneous for the treatment of canine brucellosis. In this assay 26 asymptomatic dogs were used, infected naturally with *Brucella canis* that were diagnosed in an opened canine population of a veterinary doctor's office, which numbered them by order of positive diagnosis and they were included alternatively in groups A and B according to the moment at which its positivity was detected. With this disposition two groups of 13 dogs each one were integrated. It was not consider race, age, sex, nor previous vaccinations, patients were selected on the basis of the following criteria:
1. Of inclusion: to demonstrate the asymptomatic infection by *Brucella canis* by the positive test of agglutination in plate.
2. Of exclusion: not to have proprietor, to suffer severe undernourishment, to suffer of other diseases or concurrent pathologies.
3. Of elimination: suspension of the treatment, death during the development of the clinical assay, deviation of the patient, not to have to the patient under ceiling and shelter, and not to supplemented the prescribed diet to it.

For each one of patients including in the assays, the energetic necessities were calculated (with base on the formula [corporal weight X 30 + 70] x 2), which were contributed with dry pelletized commercial food. The dog was maintained under ceiling and shelter 24 hrs at day while the treatment was running, allowing single momentary exits for the excretion of fecal and tinkles dejections.

For the elaboration of DLE from tissue, spleens of sacrificed dogs were used as described in example no. 2. After obtained the extracts, these were standardized to a concentration equivalent to 1.5 X 10⁶ leukocytes by dose, according to example no. 7. To dogs including in group A, DLE was applied to a dose equivalent to 1.5 X 10⁶ leukocytes by Kg of corporal weight via subcutaneous each 48 hrs. by 30 days. To group B it was applied a treatment with minocycline and streptomycin by three weeks. The discrimination criteria of success or failure of the treatment were negative or positive to the agglutination test in plate for *Brucella cannis* to the 45 later days of the beginning of the clinical assay.

Results obtained, after the treatment, are in the following table:

| **Group** | **DLE dose*** | **Positive**** |
|---|---|---|
| A | 1.5 X 10⁶ | 7.69 |
| B | - | 100 |

| | | |
|---|---|---|
| * By Kg of weight **To the test of agglutination in plate expressed in percentage. | | |

After evaluation of results as indicated in example no. 10, under the design of this clinical assay it is possible to be affirmed that when using DLE of sensitized canine origin for the treatment of the canine brucellosis, there is a highly significant statistical difference between groups A and B (p <0,01).

### Example 20. Treatment of canine coccidiosis with DLE from spleen of no sensitized dog.

This example corresponds to a prospective experimental assay that describes the use of DLE obtained from spleen of no sensitized dogs to a dose equivalent to 1.5 X 10⁶ leukocytes by Kg of weight with intervals of each application of 48 hrs via subcutaneous for the treatment of canine coccidiosis. In this assay 38 dogs infected naturally with *Isospora canis* were used were diagnosed, in a spread happened in a deposit of German sheepdogs. Two groups with 19 puppies each one were integrated, denominated A and B. No considered race, age, sex, nor previous vaccinations and patients were selected on the basis of the following criteria:
1. Of inclusion: to present diarrheic picture with evolution no greater to seven days, to demonstrate the infection by *Isospora canis* by coproparasitoscopic examination by flotation technique, age between 2 and 6 months.
2. Of exclusion: not to have proprietor, to suffer severe undernourishment, to suffer of dehydration moderate to severe, to suffer of other diseases or concurrent pathologies.
3. Of elimination: suspension of the treatment, increase of the diarrhea with severe dehydration, death during the development of the clinical assay, not to suplementar the prescribed diet to it.

For each one of patients including in the assays, the energetic necessities were calculated (with base on the formula [corporal weight X 30 + 70] x 2), which were contributed with dry pelletized commercial food. The dog was maintained under ceiling and shelter 24 hrs at day while the treatment was running, allowing single momentary exits for the excretion of fecal and tinkles dejections.

For the elaboration of DLE from tissue, spleens of sacrificed dogs were used as described in example no. 2. After obtained the extracts, these were standardized to a concentration equivalent to 1.5 X 10⁶ leukocyte by dose, according to example no. 7. To dogs including in group A, was applied a dose equivalent to 1.5 X 10⁶ leukocytes by Kg of corporal weight via subcutaneous each 48 hrs. by 10 days. To group B it was applied a treatment with sulfamerazine and trimethoprim by 10 days. The discrimination criteria of success or failure of the treatment were negative or positive to the serial test of three samples by the coproparasitoscopic examination using the flotation technique, the days 11, 13 and 15 later from the beginning of the clinical assay.

Results obtained, after the treatment, are in the following table:

| **Group** | **DLE dose*** | **Positive**** |
|---|---|---|
| A | 1.5 X 10⁶ | 32 |
| B | - | 100 |

| | | |
|---|---|---|
| * By Kg of weight **To the serial test expressed in percentage. | | |

After evaluation of results as indicated in example no. 10, under the design of this clinical assay it is possible to be affirmed that exist significant statistical differences between groups under study (p<0.05) when using DLE of not sensitized canine origin for the treatment of the canine coccidiosis.

### Example 21. Treatment of equine leptospirosis with DLE from blood of horse sensitized.

This example corresponds to a prospective, cross-sectional and observational clinical assay that describes the use of DLE obtained from blood of horses sensitized to a dose equivalent to 1.5 X 10⁶ leukocytes by Kg of weight with intervals of each application of 48 hrs via subcutaneous for the treatment of equine leptospirosis. In this assay 30 horses pure English blood were used, infected naturally with *Leptospira interrogans* that were detected in the block of the Police equestrian group, which numbered themselves by positivity order and they were included alternatively into groups A and B according to the moment at which they appeared for his attention. With this disposition two groups of 15 horses each one were integrated. Patients were selected according to the described in example no. 8 and did not consider race, age, sex, nor previous vaccinations. Each one of patients including in the assays, was fed with pelletized dry commercial food. For the elaboration of DLE were used 15 blood liters from three adult Creole horses, healthy clinically, not sensitized originating from a Rastro market. The extracts were obtained according to example no. 6. After obtained the extracts, these were standardized to a concentration equivalent to 1.5 X 10⁶ leukocytes by dose, according to example no. 7.

To the horses including in group A, was applied DLE to a dose equivalent to 1.5 X 10⁶ leukocytes by Kg of corporal weight via subcutaneous each 48 hrs. by 15 days. To group B it was applied procainic penicillin G (4,000,000/U) and streptomycin (22 mg/Kg) each 24 hrs. by 15 days.

The clinical signs were described from the assay beginning and every 7 days, of the following way:
**0,** when there are no signs.
**1,** when the patient presented only single prodromics signs.
**2,** when the specific signs were limited to vomit, diarrhea, poliury, hematury.
**3,** when ictericia was observed, hematury, diarrhea with melena, hematemesys.

Results obtained, after the treatment, are in the following tables:

| **Clinical valuation group A** | | | | |
|---|---|---|---|---|
| **Horse** | **Initial evaluation** | **Day 7** | **Day 14** | **Day 21** |
| 1 | 2 | 1 | 0 | 0 |
| 2 | 2 | 0 | 0 | 0 |
| 3 | 3 | 1 | 0 | 0 |
| 4 | 2 | 1 | 1 | 0 |
| 5 | 2 | 1 | 0 | 0 |
| 6 | 3 | 2 | 1 | 0 |
| 7 | 2 | 1 | 0 | 0 |
| 8 | 2 | 1 | 1 | 0 |
| 9 | 2 | 2 | 1 | 0 |

| **Clinical valuation group A** | | | | |
|---|---|---|---|---|
| **Horse** | **Initial evaluation** | **Day 7** | **Day 14** | **Day 21** |
| 10 | 2 | 1 | 0 | 0 |
| 11 | 2 | 1 | 1 | 0 |
| 12 | 2 | 0 | 0 | 0 |
| 13 | 2 | 0 | 0 | 0 |
| 14 | 3 | 2 | 0 | 0 |
| 15 | 2 | 2 | 1 | 0 |

| **Clinical valuation group B** | | | | |
|---|---|---|---|---|
| **Horse** | **Initial evaluation** | **Day 7** | **Day 14** | **Day 21** |
| 1 | 2 | 2 | 1 | 0 |
| 2 | 2 | 0 | 0 | 0 |
| 3 | 2 | 1 | 0 | 0 |
| 4 | 3 | 2 | 0 | 0 |
| 5 | 3 | 1 | 0 | 0 |
| 6 | 2 | 0 | 0 | 0 |
| 7 | 2 | 1 | 1 | 0 |
| 8 | 2 | 1 | 1 | 0 |
| 9 | 9 | 1 | 0 | 0 |
| 10 | 2 | 1 | 0 | 0 |
| 11 | 2 | 2 | 1 | 0 |
| 12 | 2 | 1 | 0 | 0 |
| 13 | 2 | 1 | 0 | 0 |
| 14 | 2 | 1 | 0 | 0 |
| 15 | 2 | 2 | 1 | 0 |

Also, for these groups it was made a serologic analysis at the beginning of the treatment as well as another 21 days later to detect antibodies anti-*Leptospira* by micro agglutination technique with alive antigen.

Results of this test are next:

| **Titers of anti-Leptospira antibodies. Group A** | | | |
|---|---|---|---|
| **Horse** | **Initial titer** | **21 Days titer** | **Sero-variety** |
| 1 | 1/100 | - | *L.ausbalis* |
| 2 | 1/200 | - | *L. grippotyphosa* |
| 3 | 1/800 | - | *L. grippotyphosa* |
| 4 | 1/200 | - | *L. pomona* |
| 5 | 1/100 | - | *L. grippotyphosa* |
| 6 | 1/800 | 1/200 | *L.pomona* |

| **Titers of anti-Leptospira antibodies. Group A** | | | |
|---|---|---|---|
| **Horse** | **Initial titer** | **21 Days titer** | **Sero-variety** |
| 7 | 1/100 | - | *L. pomona* |
| 8 | 1/200 | - | *L. grippotyphosa* |
| 9 | 1/100 | - | *L.australis* |
| 10 | 1/100 | - | *L. australis* |
| 11 | 1/200 | - | *L. pomona* |
| 12 | 1/100 | - | *L. pomona* |
| 13 | 1/200 | 1/50 | *L. pomona* |
| 14 | 1/400 | 1/50 | *L. australis* |
| 15 | 1/100 | - | *L. pomona* |

| **Titers of anti-Leptospira antibodies. Group B** | | | |
|---|---|---|---|
| **Horse** | **Initial titer** | **21 Days titer** | **Sero-variety** |
| 1 | 1/200 | - | *L. australis* |
| 2 | 1/200 | - | *L. australis* |
| 3 | 1/200 | - | *L. australis* |
| 4 | 1/400 | 1/100 | *L. pomona* |
| 5 | 1/200 | - | *L. grippotyphosa* |
| 6 | 1/100 | - | *L. pomona* |
| 7 | 1/100 | - | *L pomona* |
| 8 | 1/100 | - | *L. grippotyphosa* |
| 9 | 1/400 | 1/50 | *L. gropotyphosa* |
| 10 | 1/100 | - | *L.australis* |
| 11 | 1/100 | 1/50 | *L. australis* |
| 12 | 1/100 | - | *L pomona* |
| 13 | 1/100 | - | *L pomona* |
| 14 | 1/200 | 1/50 | *L. australis* |
| 15 | 1/100 | - | *L. grippotyphosa* |

The obtained results were put under a statistical evaluation. In the case of the valuation of the clinical signs the test of Kruskall-Wallis was used and for the case of the microagglutination the t of Student test. After evaluation of results under the design of this clinical assay it is possible to be affirmed that there is no statistical difference between groups under study (p >0.05) when using DLE from not sensitized equine origin and the treatment with antimicrobial compounds. In other words both treatments were equally effective against the equine leptospirosis.

### Example 22. Treatment of Equine influenza with DLE from spleen of sensitized horse.

This example corresponds to a prospective, cross-sectional and observational clinical assay that describes to the use of DLE obtained from spleen of sensitized horses to a dose equivalent to 1.5 X 10⁶ leukocytes by Kg of weight with intervals of each application of 48 hrs via subcutaneous for the treatment of equine influenza. In this assay 20 Appaloosa race horses, were used infected naturally with equine influenza orthomyxovirus, in a spread of a deposit. That equine population was divided in 4 males and 16 females with ages between two and twenty years; with these animals two groups were formed with ages and similar sexes, to which group denominated group A and group B. It was not considered race, age, sex, nor previous vaccinations, and patients selected on the basis of the following criteria:
1. Of inclusion: to present clinical signs of influenza, to demonstrate the infection by Orthomyxovirus by sero-neutralization test.
2. Of exclusion: to suffer severe undernourishment, to suffer moderate dehydration to severe, to suffer other diseases or concurrent pathologies.
3. Of elimination: suspension of the treatment, death during the development of the clinical assay.

To each one of patients including in the assays, was fed with dry pelletized commercial food and treat alfalfa.

For the elaboration of DLE were used three spleens from Creole, adult, clinically healthy and not sensitized horses, originating of a sign. The extracts were obtained according to example no. 2. After obtained the extracts, these were standardized to a concentration equivalent to 1.5 X 10⁶ leukocytes by dose, according to example no. 7.

To the horses including in group A, DLE was applied to a dose equivalent to 1.5 X 10⁶ leukocytes by Kg of corporal weight via subcutaneous each 48 hrs. by 15 days. To group B it was applied saline physiological solution by the same via and to a volume equivalent to the used for DLE.

The success criteria on the treatment consisted of the clinical disappearance of all the indicative signs to suffer equine influenza. The failure criterion was the persistence of a single clinical sign of the disease. Two clinical valuations on groups under study were made, the first at the seven days from de beginning and other to the 15 days.

Results obtained, after the treatment, are in the following table:

| **Group** | **DLE dose*** | **Clinical recovery**** | **Morbidity** | **Clinical recovery**** | **Morbidity** |
|---|---|---|---|---|---|
| | | First week | | Second week | |
| A | 1.5 X 10⁸ | 100 | 0 | 100 | 0 |
| B | - | 10 | 90 | 50 | 50 |

| | | | | | |
|---|---|---|---|---|---|
| * By Kg of weight ** Disappearance of signs, expressed in percentage. | | | | | |

After evaluation of results as indicated in example no. 10, under the design of this clinical assay it can affirm that exist significant statistical differences on favor to group of horses treated with DLE (p<0.05) with respect to group non treated, when using DLE of not sensitized equine origin to the treatment of equine influenza.

### Example 23. Treatment of porcine Aujesky's disease with DLE from blood of pig sensitized.

This example corresponds to a prospective, cross-sectional and observational clinical assay that describes the use of DLE obtained from blood of pigs sensitized to a dose equivalent to 1.5 X 10⁶ leukocytes by Kg of weight with intervals of each application of 48 hrs via subcutaneous for the treatment of pigs Aujesky's disease. In this assay 20 Landrace race pigs of 6 weeks of age free of specific antibodies against the Aujesky's disease were used. Later to three days of acclimatization, in cages closed (not outdoors) collective with cement floor and partition walls, under temperatures average of 20°C and relative humidity from 40 to 50%, animals were grouped in two groups of 15 pigs each one, which were denominated group A and group B. It was not considered race, age, sex, nor previous vaccinations and patients were selected on the basis of the following criteria:
1. Of inclusion: to be clinically healthy, not to present antibodies against the Aujesky's disease.
2. Of exclusion: to suffer undernourishment, to suffer other diseases or concurrent pathologies.
3. Of elimination: suspension of the treatment, death by another cause different from the infection by Aujesky's disease.

All the pigs that reunited the criteria of inclusion in this assay were inoculated by intranasal via with 3 ml of challenge virus stock Becker strain of the Aujesky's virus (10⁷ DICC50%/ml) which propagated and titer in cells of bovine kidney (MDBK) using like minimum essential media added with 10% of bovine fetal serum. At the five post inoculation days and every time the indicative of respiratory infection and viremia (rinorrea, epifora, sneeze, cough, depression and fever) were appeared, it was administered DLE to them. For the elaboration of DLE, 15 blood liters from vaccinated healthy pigs against the Aujesky's disease with vaccine of modified active virus of the Bartha strain were used. The extracts were obtained according to example no. 5. After obtained the extracts, these were standardized to a concentration equivalent to 1.5 X 10⁶ leukocytes by dose, according to example no. 7.

Group A was treated with DLE to a dose equivalent to 1.5 X 10⁶ leukocytes by Kg of corporal weight via intramuscular each 48 hrs. by 10 days, group B received exclusively saline physiological solution in similar volumes to those of DLE of group A.

The clinical signs were described from the beginning as the analysis and each 24 hrs, on the following way:
**0** when there are no signs.
**1** when the patient presented only prodromic signs (fever, mental depression, anorexia).
**2** when the specific signs were limited to epifora, rinorrea, sneeze, cough, taquipnea.
**3** when ataxia was observed, paralysis, convulsions, estuporose postration and death.

Results obtained, after the treatment, are in the following table:

| **Severity of clinical signs per day. Group A. DLE dose* 1.5 X 10⁶** | | | | | | | | | | |
|---|---|---|---|---|---|---|---|---|---|---|
| | **Day** | | | | | | | | | |
| **Pig** | **1** | **2** | **3** | **4** | **5** | **6** | **7** | **8** | **9** | **10** |
| **1** | 1 | 1 | 1 | 2 | 2 | 2 | 0 | 0 | 0 | 0 |

| **Severity of clinical signs per day. Group A. DLE dose* 1.5 X 10⁶** | | | | | | | | | | |
|---|---|---|---|---|---|---|---|---|---|---|
| | **Day** | | | | | | | | | |
| **Pig** | **1** | **2** | **3** | **4** | **5** | **6** | **7** | **8** | **9** | **10** |
| **2** | 1 | 1 | 1 | 2 | 2 | 1 | 0 | 0 | 0 | 0 |
| **3** | 1 | 1 | 1 | 2 | 2 | 2 | 1 | 0 | 0 | 0 |
| **4** | 0 | 1 | 2 | 2 | 2 | 1 | 0 | 0 | 0 | 0 |
| **5** | 1 | 1 | 1 | 2 | 2 | 1 | 0 | 0 | 0 | 0 |
| **6** | 0 | 1 | 1 | 2 | 2 | 1 | 0 | 0 | 0 | 0 |
| **7** | 1 | 1 | 2 | 2 | 2 | 1 | 0 | 0 | 0 | 0 |
| **8** | 0 | 1 | 1 | 2 | 2 | 1 | 0 | 0 | 0 | 0 |
| **9** | 0 | 1 | 1 | 2 | 2 | 1 | 0 | 0 | 0 | 0 |
| **10** | 0 | 1 | 1 | 2 | 2 | 2 | 1 | 0 | 0 | 0 |

| | | | | | | | | | | |
|---|---|---|---|---|---|---|---|---|---|---|
| * By Kg of weight | | | | | | | | | | |

| **Severity of clinical signs per day. Group B. Without DLE dose** | | | | | | | | | | |
|---|---|---|---|---|---|---|---|---|---|---|
| | **Day** | | | | | | | | | |
| **Pig** | **1** | **2** | **3** | **4** | **5** | **6** | **7** | **8** | **9** | **10** |
| **1** | 1 | 1 | 1 | 1 | 2 | 2 | 2 | 2 | 3 | 3 |
| **2** | 0 | 1 | 2 | 2 | 2 | 2 | 3 | 3 | 3 | 3 |
| **3** | 1 | 1 | 1 | 2 | 2 | 3 | 3 | 3 | 3 | 3 |
| **4** | 0 | 1 | 1 | 2 | 2 | 2 | 3 | 3 | 3 | 3 |
| **5** | 1 | 1 | 1 | 2 | 2 | 2 | 3 | 3 | 3 | 3 |
| **6** | 1 | 1 | 1 | 1 | 2 | 2 | 2 | 2 | 3 | 3 |
| **7** | 1 | 1 | 2 | 2 | 2 | 2 | 3 | 3 | 3 | 3 |
| **8** | 1 | 1 | 1 | 2 | 2 | 3 | 3 | 3 | 3 | 3 |
| **9** | 1 | 1 | 2 | 2 | 3 | 3 | 3 | 3 | 3 | 3 |
| **10** | 0 | 1 | 1 | 2 | 2 | 2 | 3 | 3 | 3 | 3 |

For the statistical analysis the test of Kruskall-Wallis was used. The design of this clinical assay allows to affirm that there are significant statistical differences on favor of group A with respect to the B (p ≤0.01) when using DLE of sensitized pig sanguineous origin for the treatment of the Aujesky's disease.

### Example 24. Treatment of porcine atrophic rhinitis with DLE from spleen of pig sensitized.

This example corresponds to a prospective, cross-sectional and observational clinical assay that describes the use of DLE obtained from spleen of pigs sensitized to a dose equivalent to 1.5 X 10⁶ leukocytes by Kg of weight with intervals of each application of 48 hrs via subcutaneous for the treatment of atrophic rhinitis in pigs. In this assay 30 Duroc race pigs were used, infected naturally with *Bordetella bronchiseptica* and *Pasterella multocida* D toxigenic type, that were detected in a complete cycle farm. With these patients two groups of 15 pigs each one were integrated. Age, sex, nor the previous vaccinations was not taken under consideration,. So that a pig could be considered as candidate to enter to this clinical assay had to fulfill the following criteria:
1. Of inclusion: to present clinical signs of infection by *Bordetella bronchiseptica* and *Pasterelia multocida* D toxigenic type, to demonstrate the infection by *Bordetella bronchiseptica* and *Pasterella multocida* D toxygenic type, at the 10 weeks of age, by bacterial isolation.
2. Of exclusion: to suffer severe undernourishment, to suffer moderate dehydration to severe, to suffer other diseases or concurrent pathologies.
3. Of elimination: suspension of the treatment, death during the development of the clinical assay.

To all the pigs that were applied to the criteria of inclusion and exclusion in this assay were fed with pelletized dry commercial food.

For the elaboration of DLE were used 20 blood liters from 5 pigs sensitized in two occasions, 30 and 15 days before, with commercial toxoid against *Borrdetella bronchiseptica* and *Pasterella multocida* D toxigenic type (Atrobac, Lab. Pfizer). The extracts were obtained according to example no. 5 After obtained the extracts, these were standardized to a concentration equivalent to 1.5 X 10⁶ leukocytes by dose, according to example no. 7.

To the pigs including in group A DLE was applied to a dose equivalent to 1.5 X 10⁶ leukocytes by Kg of corporal weight via intramuscular each 48 hrs. by 15 days, initiating their application at the 12 weeks of life. To group B it was applied saline physiological solution with the same volume and the same frequency. To the 24 weeks the pigs were sacrificed and the braquignatia degree was evaluated and the atrophic rhinitis degrees according to the techniques described by Done.³⁵

Results obtained, after the treatment, are in the following table:

| **Group** | **DLE dose*** | **Branquignatia**** | **Rhinitis**** |
|---|---|---|---|
| A | 1.5 X 10⁶ | 1.112 ± 0.398 | 1.155 ± 0.802 |
| B | - | 1.498 ± 0.534 | 2.324 ± 1.483 |

| | | | |
|---|---|---|---|
| * By Kg of weight ** Considering the average and its standard deviation. | | | |

After evaluation of results, under the design of this one clinical assay it is possible to be affirmed that there is no statistical difference in the dimension of braquignatia between the study groups (p>0.01) when using DLE of not sensitized pig origin and the control. However highly significant differences were observed (p≤0.01) when valuing the rhinitis degrees, on favor of group of pigs treated with DLE from sensitized pig sanguineous origin compared with the pigs without treatment.

### Example 25. Treatment of porcine pneumonia with DLE from spleen of pig sensitized.

This example corresponds to a prospective, cross-sectional and observational clinical assay that describes the use of DLE obtained from spleen of pigs sensitized to a dose equivalent to 1.5 X 10⁶ leukocytes by Kg of weight with intervals of each application of 48 hrs via subcutaneous for the treatment of pneumonia by complicated Mycoplasmosis in pigs. In this assay 30 Landrace race pigs were used, infected naturally with *Mycoplasma hyopneumaniae* and *Pasterella multocida* D toxigenic type, in an acute bud of respiratory disease on complete cycle farm, in which it was diagnosed mycobacteriosis by anatomopatologic injuries and pausterelosis by microbiological culture. With the 30 pigs two groups of 15 pigs each one were integrated with ages between 4 and 12 weeks of age. It was not taken in consideration, sex, nor the previous vaccinations. So that a pig could be considered as a candidate to enter to this clinical assay had to fulfill the following criteria:
1. Of inclusion: to present clinical signs of respiratory infection, to be lodged in a corral in where another pig has died with anatomopatologic diagnosis of *Mycoplasma* hyopneumaniae and microbiological diagnosis of Pasterella multocida, to have an age between 4 and 12 weeks, not to present more than 7 days with signs of respiratory disease.
2. Of exclusion: to suffer severe undernourishment, to suffer moderate dehydration to severe, to suffer other diseases or concurrent pathologies.
3. Of elimination: suspension of the treatment, death during the development of the clinical assay.

All the pigs that were applied the criteria of inclusion and exclusion in this assay were fed with peletized dry commercial food, for the growth stage.

For the elaboration of DLE were used 10 spleens of sensitized pigs with bacterine against Mycoplasma *hyopneumaniae* and commercial toxoid against *Bordetella bronchiseptica and Pasterella multocida* D toxigenic type (Atrobac, Lab. Pfizer). The lymphoid tissue was obtained from a local sign. The extracts were obtained according to example no. 2. After obtained the extracts, these were standardized to a concentration equivalent to 1.5 X 10⁶ leukocytes by dose, according to example no. 7.

To the pigs including in group A DLE was applied to a dose equivalent to 1.5 X 10⁶ leukocytes by Kg of corporal weight via intramuscular each 48 hrs. by 10 days. To group B it was applied saline physiological solution with the same volume and with the same frequency that DLE of group A.

The clinical signs were qualify, in both groups, from the beginning of the assay and each 24 hrs. during following the 10 days, of the following way:
**0** when there are no signs.
**1** when the patient presented slight mental depression, anorexia.
**2** when the specific signs were limited to sneeze, dry cough, diarrhea.
**3** when it was observed taquipnea, cyanosis, estuporose postration and death. Results obtained, after the treatment, are in the following tables

| **Severity of clinical signs per day. Group A. DLE dose* 1.5 X 10⁶** | | | | | | | | | | |
|---|---|---|---|---|---|---|---|---|---|---|
| | **Day** | | | | | | | | | |
| **Pig** | **1** | **2** | **3** | **4** | **5** | **6** | **7** | **8** | **9** | **10** |
| **1** | 1 | 1 | 1 | 1 | 1 | 0 | 0 | 0 | 0 | 0 |
| **2** | 1 | 1 | 1 | 1 | 1 | 1 | 0 | 0 | 0 | 0 |
| **3** | 1 | 1 | 2 | 2 | 2 | 2 | 2 | 2 | 2 | 1 |
| **4** | 2 | 2 | 2 | 2 | 2 | 2 | 1 | 1 | 0 | 0 |
| **5** | 1 | 2 | 2 | 2 | 1 | 1 | 0 | 0 | 0 | 0 |
| **6** | 1 | 1 | 1 | 2 | 2 | 1 | 0 | 0 | 0 | 0 |
| **7** | 1 | 2 | 2 | 2 | 2 | 2 | 1 | 1 | 1 | 0 |
| **Severity of clinical signs per day. Group A. DLE dose* 1.5 X 10⁶** | | | | | | | | | | |
| | **Day** | | | | | | | | | |
| **Pig** | **1** | **2** | **3** | **4** | **5** | **6** | **7** | **8** | **9** | **10** |
| **8** | 2 | 2 | 2 | 2 | 2 | 1 | 1 | 1 | 1 | 1 |
| **9** | 1 | 1 | 1 | 2 | 1 | 0 | 0 | 0 | 0 | 0 |
| **10** | 2 | 2 | 2 | 2 | 2 | 1 | 1 | 1 | 0 | 0 |
| **11** | 1 | 1 | 1 | 1 | 0 | 0 | 0 | 0 | 0 | 0 |
| **12** | 1 | 1 | 1 | 1 | 1 | 0 | 0 | 0 | 0 | 0 |
| **13** | 1 | 1 | 1 | 1 | 1 | 1 | 0 | 0 | 0 | 0 |
| **14** | 2 | 2 | 2 | 2 | 2 | 2 | 1 | 1 | 1 | 1 |
| **15** | 1 | 1 | 1 | 1 | 1 | 1 | 0 | 0 | 0 | 0 |

| **Severity of clinical signs per day. Group B. Without DLE dose** | | | | | | | | | | |
|---|---|---|---|---|---|---|---|---|---|---|
| | **Day** | | | | | | | | | |
| **Pig** | **1** | **2** | **3** | **4** | **5** | **6** | **7** | **8** | **9** | **10** |
| **1** | 1 | 1 | 1 | 1 | 2 | 2 | 2 | 2 | 2 | 2 |
| **2** | 1 | 1 | 2 | 2 | 2 | 2 | 2 | 2 | 2 | 3 |
| **3** | 2 | 2 | 2 | 2 | 2 | 3 | 3 | 3 | 3 | 3 |
| **4** | 1 | 1 | 1 | 2 | 2 | 2 | 1 | 2 | 2 | 3 |
| **5** | 1 | 1 | 1 | 1 | 1 | 1 | 1 | 1 | 1 | 1 |
| **6** | 1 | 1 | 1 | 1 | 2 | 2 | 2 | 2 | 2 | 3 |
| **7** | 1 | 1 | 2 | 2 | 2 | 2 | 3 | 3 | 3 | 3 |
| **8** | 1 | 1 | 1 | 2 | 2 | 3 | 3 | 3 | 3 | 3 |
| **9** | 1 | 1 | 1 | 1 | 2 | 2 | 2 | 2 | 2 | 2 |
| **10** | 1 | 1 | 1 | 2 | 2 | 2 | 3 | 3 | 3 | 3 |
| **11** | 2 | 2 | 2 | 2 | 2 | 2 | 2 | 3 | 3 | 3 |
| **12** | 2 | 2 | 2 | 2 | 2 | 2 | 2 | 2 | 1 | 1 |
| **13** | 1 | 1 | 1 | 1 | 1 | 1 | 1 | 2 | 2 | 2 |
| **14** | 2 | 2 | 2 | 2 | 2 | 2 | 2 | 2 | 2 | 2 |
| **15** | 1 | 1 | 1 | 1 | 2 | 2 | 2 | 2 | 2 | 3 |

| | | | | | | | | | | |
|---|---|---|---|---|---|---|---|---|---|---|
| * By Kg of weight | | | | | | | | | | |

For the statistical analysis the test of Kruskall-Wallis was used. After evaluation of results, under the design of this clinical assay it is possible to be affirmed that there are statistical differences on favor group A with respect to the B (p≤0.05) when using DLE of sensitized pig sanguineous origin for the treatment of the respiratory disease by micoplasmosis and porcine pasteurelosis.

### References

1. Spinelli, SJ. 1986. Farmacologia Veterinaria y Terapeutica. Mexico: Interamericana.
2. Apple MJ. 1998. La Consulta Veterinaria en 5 Minutos. Buenos Aires: Inter-Médica.
3. Thompson H. 1998. Canine Medicine and Therapeutics. Oxford: Blackwell Science.
4. Sherding RG. 1996. Manual Clinico de Pequeñas Especies. Mexico: Interamericana.
5. Greene CE 1993. Enfermedades Infecciosas, Dogs y Gatos. Mexico: Interamericana.
6. Davenport DJ. 1993. Pediatria Veterinaria, Dogs y Gatos. México: Interamericana.
7. Swango LJ. 1995. Textbook of Veterinary Internal Medicine, Diseases of the Dog and Cat. Philadelphia: W.B. Saunders.
8. Lawrence, H.S. 1949. Proc. Soc. Exp. Biol. Med. 71: 516-522.
9. Lawrence, H.S. 1955. J. Clin. Inv. 34: 219-230.
10. Levin, A.S. 1970. Proc. Nat. Acad. Sci. 67: 821-828.
11. Ferrer Argote, V.E. 1995. Rev. Med. Hospital General de Mexico, S.S. 58: 148-150.
12. US. Pat. 4,435,384.
13. Wolf, R.E. 1978. Clin. Immunol Immunopathol 10: 292-297.
14. Steele. 1980. New Engl. J. Med. 303: 355-359.
15. Louie. 1987. Clin. Immunol Immunopath 44: 329-334.
16. McMeeking. 1990. J. Infect. Dis. 161: 108-112.
17. Estrada Parra, S. 1983. Salud Publica de Mexico. 6: 579-590.
18. Kirkpatrick, C.H. 1979. Immune Regulators in Transfer Factor. Academic. Press. New York.
19. Delgado, O. 1981. Clin. Immunol Immunopathol. 19: 351-359.
20. Mikula, I. 1992. Vet. Immunol. Immunopathol. 32: 113-124.
21. Kirkpatrick, C.H. 1993. Annal New York Acad Sci. 362:368.
22. Klesius, P.H. 1978. Clin. Immunol Immunopathol. 10: 214-221.
23. Littman, B.H. 1978. Clin. Immunol Immunopathol. 9: 97-110.
24. Klesius, P.H. 1984. Poultry Sci. 63: 1333-1337.
25. Lawrence, H.S. 1960. Ciba Foundat on Symposium on Cellular Aspects of Immunity (Boston: Little Brown). 243.
26. Lawrence, H.S. 1966.Trans. Ass. Amer. Physicians. 76: 84.
27. Baram, P. 1966. J. Immunol. 97: 407.
28. Haddad, Z.H. 1968. J. Allergy. 41:112.
29. Khan, A. 1980. Cell. Inmunol., 55: 420.
30. Gottlieb, A..A. 1982. Clin. Exp. Immunol., 50: 434.
31. Wilson, G.B., et. al. 1977. Clin Immunol Immunopathol, 8:551.
32. Kirkpatrick, C.H. 1999. US Pat 5,883,224.
33. Mikula, I. 1992. Vet. Immunol. Immunopathol. ,32: 103-112.
34. Fudenberg, H.H. 1989. Annu. Rev. Pharmacol. Toxicol. 29: 475-516.
35. Done,J.T. 1980. Vet. Annual 15: 1-7

## Claims

1. A sterile dialyzed extract of leukocytes, free of complete or viable cells and that contains molecules smaller than 12 KDal of weight, **characterized in that** it is stable in solution at 4°C during 7 to 12 months without the use of preservers and wherein the dialyzed extract is sterilized by ozone addition.

2. The extract of claim 1, **characterized in that** it contains an equivalent concentration of leukocytes of 1 X 10⁵ to 5 X 10⁹ by ml.

3. The extract of claim 1 or 2, **characterized in that** the leukocytes are from blood or lymphatic tissue of animal.

4. The extract of claim 3 **characterized in that** the lymphatic tissue is selected of thymus, lymph nodes, spleen or combinations of such.

5. The extract of anyone of claims 1 to 4 **characterized in that** it is prepared from tissues of previously sensitized animals against an etiological specific infectious agent.

6. The extract of anyone of claims 1 to 4 **characterized in that** it is prepared from no sensitized animal tissues against an etiological specific infectious agent.

7. A pharmaceutical composition **characterized in that** it comprises a therapeutically effective amount of dialyzed leukocyte extract of any one of claims 1 to 6 in a pharmaceutically acceptable vehicle.

8. The pharmaceutical composition of claim 7, **characterized in that** the dialyzed leukocyte extract is in an equivalent concentration of leukocytes of 1 X 10⁵ to 5 X 10⁹ by ml.

9. The pharmaceutical composition of claim 7 or 8, **characterized in that** it contains additionally an antibiotic.

10. The pharmaceutical composition of claim 9, **characterized in that** the antibiotic is selected from the group comprising sulfamerazine, trimetropin, minocycline, amoxicillin, gentamicin, penicillin and streptomycin.

11. A therapeutic kit for the treatment of a nonhuman animal with an infection disease, **characterized in that** it comprises 6 to 10 individual doses of the pharmaceutical composition of anyone of claims 7 to 10, where the 30% to 50% of the said individual doses are 3 to 300 times greater in the concentration of the dialyzed leukocyte extract than the remaining doses.

12. A method for obtaining the dialyzed leukocyte extract of anyone of claims 1 to 6, comprising the steps of :
Collecting the cellular tissues that contain leukocytes of a donating nonhuman animal,
Separating the leukocytes of cellular tissues obtained in a),
Lysating the leukocytes obtained in b),
Dialyzing the extract obtained in step c),
**characterized in that** the dialyzed extract obtained in step d) is sterilized by ozone addition to a concentration of 0.2 to 0.5 ppm applied by bubbling.

13. The method of claim 12, **characterized in that** ozone is added to a concentration of 0.3 ppm.

14. Use of a dialyzed leukocyte extract of anyone of claims 1 to 6 for the manufacture of a medicine for the treatment of a vertebrate non-human animal with an infection caused by a microorganism selected of the group that consists of bacteria, fungi, protozoa and virus, that includes administering to the infected non-human animal a therapeutically effective amount of the extract.

15. The use of claim 14 **characterized in that** the dialyzed leukocyte extract is administered at a dose equivalent to 1 X 106 to 5 X 106 leukocytes by Kg of corporal weight.

16. The use of claim 15, **characterized in that** the dialyzed leukocyte extract is administered at a dose equivalent to 1.5 X 106 leukocytes by Kg of corporal weight.

17. The use of any one of claims 14 to 16, **characterized in that** the dialyzed leukocyte extract is applied at an interval between doses of 8 hrs to 30 days.

18. The use of any one of claims 14 to 16, **characterized in that** the dialyzed leukocyte extract is administered via parenteral or enteral.

19. The use of claim 18, **characterized in that** the dialyzed leukocyte extract administered via subcutaneous.

20. The use of any one of claims 14 to 19, **characterized in that** the dialyzed leukocyte extract is applied in unique dose.

21. The use of claim 14, wherein the vertebrate nonhuman animal is mammalian or bird.

22. The use of claim 21, wherein the mammal are selected of group that consists of felines, canines, porcines, equines, bovines and ovines.

23. The use of claim 21, wherein the microorganism is a virus selected from α-herpes virus, β-herpes virus, α^{~} herpes virus, Morbillivirus, pneumovirus, coronavirus, papilomavirus, oncornavirus, orthomyxovirus, paramyxovirus, lentivirus and oncovirus; or wherein the microorganism is a bacterium selected from *Pasteurella, Mycoplasma, Mycobacterium, Leptospira, Brucella, Bordetella, Haemophilus, Treponema, and Campylobacter;* or wherein the microorganism is a fungus selected from *Actinomyces, Coccidioidomicosis, Histoplasma, Criptococcus, Blastomyces, Aspergillus* and *Candida;* or wherein the microorganism is a protozoa selected from *Leishmania, Entamoeba, Toxoplasma, Isospora, Erlichia, Neospora, Criptosporidium* and *Balantidium.*

24. The use of claim 21, wherein the infection caused by virus is selected from bovine infectious rinotraqueitis, ulcerative mammillitis, malignant catarrhal fever, influenza, bovine plague, parainfluenza-3, bovine sincitial virus, diarrhea neobirthday of calf, papilomatosis, bovine leucosis, equine rinoneumonitis, exanthema coital equine, equine infectious anemia, parainfluenza SV-5, herpesvirus canine, feline Rinotraqueitis, feline urolitiasis, feline infectious peritonitis, feline viral leukemia, herpes virus of the nursing pig, atrophic rhinitis, transmissible gastroenteritis, hemaglutinant encephalomyelitis, disease of the blue eye, pulmonary adenomatosis, infectious laringotraquitis, Marek's disease, bird plague, Newcastle's disease, infectious bronchitis and bird leucosis; or wherein the infection caused by bacteria is selected from hemorrhagic septicemia, contagious pleuropneumoniae, bovine mastitis, paratuberculosis, tuberculosis, leptospirosis, brucellosis, enzootic pneumonia of pigs, dysentery pig, campylobacteriosis, chronic respiratory disease, infectious sinovitis and bird cholera; or wherein the infection caused by fungi is selected from actinomicosis, coccidioidomicosis, histoplasmosis, criptococosis, blastomicosis, aspergillosis and candidiasis; or wherein the infection caused by protozoa is selected from toxoplasmosis, leishmaniasis, balantidiasis, amebiasis, coccidiosis, erlichiosis and neosporosis.

25. The use of claim 21, wherein the extract is prepared from tissues of a nonhuman animal of the same specie or from other different specie than the nonhuman animal that receive the treatment.

26. The use of claim 14, **characterized in that** the infection caused by virus is selected from the group consisting of canine distemper and Aujesky's disease.

27. The use of claim 26, **characterized in that** the dialyzed leukocyte extract is administered at a dose equivalent to 1 X 10⁵ to 5 X 10⁹ leukocytes by Kg of corporal weight; or **in that** the dialyzed leukocyte extract is administered at a dose equivalent to 1.5 X 106 leukocytes by Kg of corporal weight.

28. The sterile dialyzed extract of any one of claims 1 to 6 for use in treating a vertebrate non-human animal with an infection caused by a microorganism selected of the group that consists of bacteria, fungi, protozoa and virus, that includes administering to the infected non-human animal a therapeutically effective amount of the extract.

29. The sterile dialyzed extract for use according to claim 28 **characterized in that** the extract is administered at a dose equivalent to 1 X 106 to 5 X 106 leukocytes by Kg of corporal weight; or **in that** the extract is administered at a dose equivalent to 1,5 X 106 leukocytes by Kg of corporal weight.

30. The sterile dialyzed extract for use according to any one of claims 28 to 29 for treating a vertebrate non-human animal with an infection caused by a microorganism selected of the group that consists of canine distemper and Aujesky's disease.

31. Sterile dialyzed extract of leukocytes, free of complete or viable cells containing molecules smaller than 12 KDal of weight for use in treating a vertebrate non-human animal with an infection caused by a microorganism selected of the group that consists of canine distemper and Aujesky's disease.

## Patentansprüche

1. Steriler dialysierter Leukozytenextrakt, welcher frei von vollständigen oder lebensfähigen bzw. wachstumsfähigen Zellen ist, und welcher Moleküle mit einem Gewicht kleiner als 12 KDal (Kilo-Dalton) aufweist, **dadurch gekennzeichnet, dass** er in einer Lösung bei 4°C 7 bis 12 Monate haltbar ist, ohne dass Konservierungsmittel verwendet werden, und dass der dialysierte Extrakt durch die Zugabe von Ozon sterilisiert bzw. keimfrei gemacht wird.

2. Extrakt nach Anspruch 1, **dadurch gekennzeichnet, dass** er eine äquivalente Konzentration an Leukozyten von 1 x 10⁵ bis 5 x 10⁹ pro ml enthält.

3. Extrakt nach Anspruch 1 oder 2, **dadurch gekennzeichnet, dass** die Leukozyten aus Blut oder lymphatischem Gewebe von Tieren stammen.

4. Extrakt nach Anspruch 3, **dadurch gekennzeichnet, dass** das lymphatische Gewebe aus Thymus, Lymphknoten, Milz oder Kombinationen daraus ausgewählt wird.

5. Extrakt nach einem der Ansprüche 1 bis 4, **dadurch gekennzeichnet, dass** er aus Geweben von Tieren bereitet wird, welche zuvor für einen ursächlichen, spezifischen Infektionserreger sensibilisiert worden sind.

6. Extrakt nach einem der Ansprüche 1 bis 4, **dadurch gekennzeichnet, dass** er aus Geweben von Tieren bereitet wird, welche zuvor nicht für einen ursächlichen, spezifischen Infektionserreger sensibilisiert worden sind.

7. Pharmazeutische Zusammensetzung bzw. Rezeptur, welche **dadurch gekennzeichnet ist, dass** sie eine therapeutisch wirksame Menge an dialysiertem Leukozytenextrakt nach einem der Ansprüche 1 bis 6 in einem pharmazeutisch zulässigen Trägerstoff aufweist.

8. Pharmazeutische Zusammensetzung bzw. Rezeptur nach Anspruch 7, **dadurch gekennzeichnet, dass** der dialysierte Leukozytenextrakt in einer entsprechenden Leukozyten-Konzentration von 1 x 10⁵ bis 5 x 10⁹ pro ml vorliegt.

9. Pharmazeutische Zusammensetzung bzw. Rezeptur nach Anspruch 7 oder 8, **dadurch gekennzeichnet, dass** sie zusätzlich ein Antibiotikum enthält.

10. Pharmazeutische Zusammensetzung bzw. Rezeptur nach Anspruch 9, **dadurch gekennzeichnet, dass** das Antibiotikum aus der Gruppe bestehend aus Sulfamerazin, Trimetropin, Minocyclin, Amoxicillin, Gentamicin, Penicillin und Streptomycin ausgewählt wird.

11. Therapeutische Ausrüstung für die Behandlung eines nichtmenschlichen Lebewesens mit einer Infektionskrankheit, **dadurch gekennzeichnet, dass** die Ausrüstung 6 bis 10 individuelle Dosen der pharmazeutischen Zusammensetzung bzw. Rezeptur nach einem der Ansprüche 7 bis 10 aufweist, wobei 30% bis 50% dieser individuellen Dosen die 3- bis 300-fache Konzentration des dialysierten Leukozytenextrakts im Ver- gleich zu den übrigen Dosen aufweist.

12. Verfahren zum Erhalt des dialysierten Leukozytenextrakts nach einem der Ansprüche 1 bis 6, wobei das Verfahren die folgenden Schritte aufweist:
a. Sammeln bzw. Bestimmen der Zellgewebe, welche Leukozyten eines nicht menschlichen Spender-Lebewesens enthalten;
b. Trennen der Leukozyten aus den in a) erhaltenen Zellgeweben;
c. Lysieren der in b) erhaltenen Leukozyten;
d. Dialysieren des in Schritt c) erhaltenen Extrakts, **dadurch gekennzeichnet, dass** der in Schritt d) erhaltene dialysierte Extrakt durch Ozonzugabe zu einer Konzentration von 0,2 bis 0,5 ppm sterilisiert wird, wobei die Ozonzugabe durch Blasenbildung erfolgt.

13. Verfahren nach Anspruch 12, **dadurch gekennzeichnet, dass** das Ozon einer Konzentration von 0,3 ppm zugesetzt wird.

14. Verwendung eines dialysierten Leukozytenextrakts nach einem der Ansprüche 1 bis 6 zur Herstellung einer Medizin für die Behandlung eines nichtmenschlichen Wirbeltiers mit einer Infektion, welche durch einen Mikroorganismus verursacht wird, welcher aus der Gruppe bestehend aus Bakterien, Pilzen, Einzellern bzw. Protozoen und Viren ausgewählt wird, wobei die Behandlung die Verabreichung einer therapeutisch wirksamen Menge des Extrakts an das infizierte nichtmenschliche Lebewesen einschließt.

15. Verwendung nach Anspruch 14, **dadurch gekennzeichnet, dass** der dialysierte Leukoztytenextrakt in einer Dosis verabreicht wird, welche 1 x 10⁶ bis 5 x 10⁶ Leukozyten pro Kg Körpergewicht entspricht.

16. Verwendung nach Anspruch 15, **dadurch gekennzeichnet, dass** der dialysierte Leukozytenextrakt in einer Dosis verabreicht wird, welche 1,5 x 10⁶ Leukozyten pro Kg Körpergewicht entspricht.

17. Verwendung nach einem der Ansprüche 14 bis 16, **dadurch gekennzeichnet, dass** der dialysierte Leukozytenextrakt in einem Zeitintervall zwischen den Dosen von 8 Stunden bis 30 Tagen angewendet wird.

18. Verwendung nach einem der Ansprüche 14 bis 16, **dadurch gekennzeichnet, dass** der dialysierte Leukozytenextrakt parenteral oder enteral verabreicht wird.

19. Verwendung nach Anspruch 18, **dadurch gekennzeichnet, dass** der dialysierte Leukozytenextrakt subkutan verabreicht wird.

20. Verwendung nach einem der Ansprüche 14 bis 19, **dadurch gekennzeichnet, dass** der dialysierte Leukozytenextrakt in einer einzigen Dosis verabreicht wird.

21. Verwendung nach Anspruch 14, **dadurch gekennzeichnet, dass** das nichtmenschliche Wirbeltier ein Säugetier oder ein Vogel ist.

22. Verwendung nach Anspruch 21, **dadurch gekennzeichnet, dass** das Säugetier aus einer Gruppe ausgewählt wird, welche Katzen, Hunde, Schweine, Pferde, Rinder und Schafe einschließt.

23. Verwendung nach Anspruch 21, **dadurch gekennzeichnet, dass** der Mikroorganismus ein Virus ist, welches aus a-Herpesvirus, b-Herpesvirus, g-Herpesvirus, Morbillivirus, Pneumovirus, Coronavirus, Papilomavirus, Oncornavirus, Orthomyxovirus, Paramyxovirus, Lentivirus und Oncovirus ausgewählt wird; oder dass der Mikroorganismus eine Bakterie ist, welche aus den Gattungen Pasteurella, Mycoplasma, Mycobakterien, Leptospira, Brucella, Bordetella, Haemophilus, Treponema und Campylobacter ausgewählt wird; oder dass der Mikroorganismus ein Pilz ist, welcher aus den Gattungen Actinomyces, Kokzidioidomykose, Histoplasma, Criptococcus, Blastomyces, Aspergillus und Candida ausgewählt wird; oder dass der Mikroorganismus ein Einzeller bzw. Protozoon ist, welches aus den Gattungen Leishmania, Enta-moeba, Toxoplasma, Isospora, Ehrlichia, Neospora, Cryptosporidium und Balantidium ausgewählt wird.

24. Verwendung nach Anspruch 21, **dadurch gekennzeichnet, dass** die von dem Virus verursachte Infektion aus der folgenden Gruppe ausgewählt wird: Bovine infektiöse Rhinotraqueitis, eiternde Mammilitis, bösartiges Katarrhalfieber, Influenza, Rinderpest, Parainfluenza 3, bovines Syncytial-Virus; Kälberdurchfall nach Geburt; Papilomatose, bovine Leukose, equine Rhinopneumonitis, equines Coital Exanthem, equine infektiöse Anämie, Parainfluenza SV-5, canines Herpesvirus, feline Rinotraqueitis, feline Urolitiase, feline infektiöse Peritonitis, feline Virusleukämie, Herpesvirus beim säugenden Schwein, atrophe Rhinitis, übertragbare Gastroenteritis, hämagglutinierende Encephalomyelitis, Blaue-Augen-Krankheit, Lungen-Adenomatose, infektiöse Laringotraquitis, Marek-Krankheit, Vögelpest, Newcastle Krankheit, infektiöse Bronchitis und Vögelleukose; oder dass die von Bakterien verursachte Infektion aus folgender Gruppe ausgewählt wird: hämorrhagische Septikämie, übertragbare Pleuropneumonie, bovine Mastitis, Paratuberkulose, Tuberkulose, Leptospirose, Brucellose, enzootische Lungenentzündung bei Schweinen, Durchfall beim Schwein, Campylobakteriose, chronische Atemwegserkrankung, infektiöse Synovitis und Vögelcholera; oder dass die durch Pilze verursachte Infektion aus der Gruppe bestehend aus Actinomykose, Kokzidioidomykose, Histoplasmose, Kryptokokkose, Blastomykose, Aspergillose und Kandidose ausgewählt wird; oder dass die durch Protozoen bzw. Einzeller verursachte Infektion aus der Gruppe bestehend aus Toxoplasmose, Leishmaniose, Balantidiose, Amöbiasis, Kokzidiose, Ehrlichiose und Neosporose ausgewählt wird.

25. Verwendung nach Anspruch 21, **dadurch gekennzeichnet, dass** der Extrakt aus Geweben eines nichtmenschlichen Lebenwesens derselben Spezies oder einer anderen unterschiedlichen Spezies als dem nichtmenschlichen Lebenwesen, welches die Behandlung erhält, bereitet wird.

26. Verwendung nach Anspruch 14, **dadurch gekennzeichnet, dass** die von dem Virus verursachte Infektion aus der Gruppe bestehend aus Hundestaupe und Aujeskysche Krankheit ausgewählt wird.

27. Verwendung nach Anspruch 26, **dadurch gekennzeichnet, dass** der dialysierte Leukozytenextrakt in einer Dosis verabreicht wird, welche 1 x 10⁵ bis 5 x 10⁹ Leukozyten pro Kg an Körpergewicht entspricht; oder dass der dialysierte Leukozytenextrakt in einer Dosis verabreicht wird, welche 1,5 x 10⁶ Leukozyten pro Kg an Körpergewicht entspricht.

28. Steriler dialysierter Extrakt nach einem der Ansprüche 1 bis 6 zur Verwendung bei der Behandlung eines nichtmenschlichen Wirbeltiers mit einer Infektion, welche durch einen Mikroorganismus verursacht wird, welcher aus der Gruppe bestehend aus Bakterien, Pilzen, Protozoen und Viren ausgewählt wird, wobei die Behandlung die Verabreichung einer therapeutisch wirksamen Menge des Extrakts an das infizierte nichtmenschliche Lebewesen einschließt.

29. Steriler dialysierter Extrakt zur Verwendung nach Anspruch 28, **dadurch gekennzeichnet, dass** der Extrakt in einer Dosis verabreicht wird, welche 1 x 10⁶ bis 5 x 10⁶ Leukozyten pro Kg an Körpergewicht entspricht; oder dass der Extrakt in einer Dosis verabreicht wird, welche 1,5 x 10⁶ Leukozyten pro Kg an Körpergewicht entspricht.

30. Steriler dialysierter Extrakt zur Verwendung nach einem der Ansprüche 28 bis 29 zur Behandlung eines nichtmenschlichen Wirbeltiers mit einer Infektion, welche durch einen Mikroorganismus verursacht wird, welcher aus der Gruppe bestehend aus Hundestaupe und Aujeskysche Krankheit ausgewählt wird.

31. Steriler dialysierter Leukozytenextrakt, welcher frei von vollständigen oder lebensfähigen bzw. wachstumsfähigen Zellen ist, wobei die Zellen Moleküle mit einem Gewicht kleiner als 12 KDal zur Verwendung bei der Behandlung von nichtmenschlichen Wirbeltieren mit einer durch einen Mikroorganismus verursachten Mikroorganismus enthalten, wobei die Infektion aus der Gruppe bestehend aus Hundestaupe und Aujeskysche Krankheit ausgewählt wird.

## Revendications

1. Extrait dialysé stérile de leucocytes, dépourvu de cellules complètes ou viables et qui contient des molécules plus petites que 12 kDa de poids, **caractérisé en ce qu'**il est stable en solution à 4° C pendant 7 à 12 mois sans l'utilisation de conservateurs et où l'extrait dialysé est stérilisé par addition d'ozone.

2. Extrait selon la revendication 1, **caractérisé en ce qu'**il contient une concentration équivalente de leucocytes de 1 x 10⁵ à 5 x 10⁹ par ml.

3. Extrait selon la revendication 1 ou 2, **caractérisé en ce que** les leucocytes proviennent du sang ou de tissu lymphatique d'animal.

4. Extrait selon la revendication 3, **caractérisé en ce que** le tissu lymphatique est choisi parmi le thymus, les ganglions lymphatiques, la rate ou des combinaisons de ceux-ci.

5. Extrait selon l'une quelconque des revendications 1 à 4, **caractérisé en ce qu'**il est préparé à partir de tissus d'animaux sensibilisés au préalable contre un agent infectieux étiologique spécifique.

6. Extrait selon l'une quelconque des revendications 1 à 4, **caractérisé en ce qu'**il est préparé à partir de tissus d'animaux non sensibilisés contre un agent infectieux étiologique spécifique.

7. Composition pharmaceutique **caractérisée en ce qu'**elle comprend une quantité thérapeutiquement efficace d'extrait leucocytaire dialysé selon l'une quelconque des revendications 1 à 6 dans un véhicule pharmaceutiquement acceptable.

8. Composition pharmaceutique selon la revendication 7, **caractérisée en ce que** l'extrait leucocytaire dialysé est dans une concentration équivalente de leucocytes de 1 x 10⁵ à 5 x 10⁹ par ml.

9. Composition pharmaceutique selon la revendication 7 ou 8, **caractérisée en ce qu'**elle contient en outre un antibiotique.

10. Composition pharmaceutique selon la revendication 9, **caractérisée en ce que** l'antibiotique est choisi dans le groupe comprenant la sulfamérazine, le triméthoprime, la minocycline, l'amoxicilline, la gentamicine, la pénicilline et la streptomycine.

11. Kit thérapeutique destiné au traitement d'un animal non humain souffrant d'une maladie infectieuse, **caractérisé en ce qu'**il comprend 6 à 10 doses individuelles de la composition pharmaceutique selon l'une quelconque des revendications 7 à 10, où 30 % à 50 % desdites doses individuelles sont 3 à 300 fois supérieures en concentration d'extrait leucocytaire dialysé que les doses restantes.

12. Procédé d'obtention de l'extrait leucocytaire dialysé selon l'une quelconque des revendications 1 à 6, comprenant les étapes consistant à :
recueillir les tissus cellulaires qui contiennent des leucocytes d'un animal non humain donneur,
séparer les leucocytes des tissus cellulaires obtenus en a),
lyser les leucocytes obtenus en b),
dialyser l'extrait obtenu dans l'étape c),
**caractérisé en ce que** l'extrait dialysé obtenu dans l'étape d) est stérilisé par addition d'ozone à une concentration de 0,2 à 0,5 ppm appliquée par bouillonnement.

13. Procédé selon la revendication 12, **caractérisé en ce que** l'ozone est ajoutée à une concentration de 0,3 ppm.

14. Utilisation d'un extrait leucocytaire dialysé selon l'une quelconque des revendications 1 à 6 pour la fabrication d'un médicament destiné au traitement d'un animal vertébré non humain souffrant d'une infection provoquée par un micro-organisme choisi dans le groupe qui est constitué de bactéries, de champignons, de protozoaires et de virus, qui comprend l'administration à l'animal non humain infecté d'une quantité thérapeutiquement efficace de l'extrait.

15. Utilisation selon la revendication 14, **caractérisée en ce que** l'extrait leucocytaire dialysé est administré à une dose équivalente à 1 x 10⁶ à 5 x 10⁶ leucocytes par kg de poids corporel.

16. Utilisation selon la revendication 15, **caractérisée en ce que** l'extrait leucocytaire dialysé est administré à une dose équivalente à 1,5 x 10⁶ leucocytes par kg de poids corporel.

17. Utilisation selon l'une quelconque des revendications 14 à 16, **caractérisée en ce que** l'extrait leucocytaire dialysé est appliqué à un intervalle entre les doses de 8 h à 30 jours.

18. Utilisation selon l'une quelconque des revendications 14 à 16, **caractérisée en ce que** l'extrait leucocytaire dialysé est administré par voie parentérale ou entérale.

19. Utilisation selon la revendication 18, **caractérisée en ce que** l'extrait leucocytaire dialysé est administré par voie sous-cutanée.

20. Utilisation selon l'une quelconque des revendications 14 à 19, **caractérisée en ce que** l'extrait leucocytaire dialysé est appliqué en une dose unique.

21. Utilisation selon la revendication 14, où l'animal vertébré non humain est un mammifère ou un oiseau.

22. Utilisation selon la revendication 21, où le mammifère est choisi dans le groupe qui est constitué de félins, de canidés, de porcidés, d'équidés, de bovins et d'ovins.

23. Utilisation selon la revendication 21, où le micro-organisme est un virus choisi parmi le virus de l'herpès α, le virus de l'herpès β, le morbillivirus, le pneumovirus, le coronavirus, le papillomavirus, l'oncornavirus, l'orthomyxovirus, le paramyxovirus, le lentivirus et l'oncovirus ; ou bien où le micro-organisme est une bactérie choisie parmi *Pasteurella, Mycoplasma, Mycobacterium, Leptospira, Brucella, Bordetella, Haemophilus, Treponema* et *Campylobacter;* ou bien où le micro-organisme est un champignon choisi parmi *Actinomyces, Coccidioidomicosis, Histoplasma, Cryptococcus, Blastomyces, Aspergilles* et *Candida ;* ou bien où le micro-organisme est un protozoaire choisi parmi *Leishmania, Entamoeba, Toxoplasma, Isospora, Erlichia, Neospora, Cryptosporidium* et *Balantidium.*

24. Utilisation selon la revendication 21, où l'infection provoquée par un virus est choisie parmi une rhinotrachéite infectieuse bovine, une thélite ulcérative, une fièvre catarrhale maligne, la grippe, la peste bovine, le para-influenza 3, le virus syncytial bovin, la diarrhée néonatale du veau, la papillomatose, la leucose bovine, la rhinopneumonie équine, l'exanthème coïtal équin, l'anémie infectieuse équine, le para-influenza SV-5, l'herpèsvirus canin, la rhinotrachéite féline, l'urolithiase féline, la péritonite infectieuse féline, la leucémie virale féline, l'herpèsvirus de la truie allaitant, la rhinite atrophique, la gastro-entérite transmissible, l'encéphalomyélite hémagglutinante, la maladie des yeux bleus, l'adénomatose pulmonaire, la laryngotrachéite infectieuse, la maladie de Marek, la peste aviaire, la maladie de Newcastle, la bronchite infectieuse et la leucose aviaire ; ou bien où l'infection provoquée par une bactérie est choisie parmi la septicémie hémorragique, la pleuropneumonie contagieuse, la mastite bovine, la paratuberculose, la tuberculose, la leptospirose, la brucellose, la pneumonie enzootique du porc, la dysenterie du porc, la campylobactériose, les maladies respiratoires chroniques, la synovite infectieuse et le choléra aviaire ; ou bien où l'infection provoquée par un champignon est choisie parmi factinomycose, la coccidioïdomycose, l'histoplasmose, la cryptococcose, la blastomycose, l'aspergillose et la candidose ; ou bien où l'infection provoquée par un protozoaire est choisie parmi la toxoplasmose, la leishmaniose, la balantidiose, l'amoebiose, la coccidiose, l'erlichiose et la néosporose.

25. Utilisation selon la revendication 21, où l'extrait est préparé à partir de tissus d'un animal non humain de la même espèce ou d'une espèce différente de l'animal non humain qui reçoit le traitement.

26. Utilisation selon la revendication 14, **caractérisée en ce que** l'infection provoquée par un virus est choisie dans le groupe constitué de la maladie de Carré et la maladie d'Aujeszky.

27. Utilisation selon la revendication 26, **caractérisée en ce que** l'extrait leucocytaire dialysé est administré à une dose équivalente à 1 x 10⁵ à 5 x 10⁹ leucocytes par kg de poids corporel ; ou **en ce que** l'extrait leucocytaire dialysé est administré à une dose équivalente à 1,5 x 10⁶ leucocytes par kg de poids corporel.

28. Extrait dialysé stérile selon l'une quelconque des revendications 1 à 6, en vue d'une utilisation dans le traitement d'un animal vertébré non humain souffrant d'une infection provoquée par un micro-organisme choisi dans le groupe qui est constitué de bactéries, de champignons, de protozoaires et de virus, qui comprend l'administration à l'animal non humain infecté d'une dose thérapeutiquement efficace de l'extrait.

29. Extrait dialysé stérile en vue d'une utilisation selon la revendication 28, **caractérisé en ce que** l'extrait est administré à une dose équivalente à 1 x 10⁶ à 5 x 10⁶ leucocytes par kg de poids corporel; ou **en ce que** l'extrait est administré à une dose équivalente à 1,5 x 10⁶ leucocytes par kg de poids corporel.

30. Extrait dialysé stérile en vue d'une utilisation selon l'une quelconque des revendications 28 à 29 pour le traitement d'un animal vertébré non humain souffrant d'une infection provoquée par un micro-organisme qui est choisie dans le groupe constitué de la maladie de Carré et la maladie d'Aujeszky.

31. Extrait dialysé stérile de leucocytes, dépourvu de cellules complètes ou viables contenant des molécules plus petites que 12 kDa de poids en vue d'une utilisation dans le traitement d'un animal vertébré non humain souffrant d'une infection provoquée par un micro-organisme qui est choisie dans le groupe constitué de la maladie de Carré et la maladie d'Aujeszky.
